(19) Europäisches Patentamt
European Patent Office
Office européen des brevets

(11) Veröffentlichungsnummer: **0 637 585 A1**

(12) **EUROPÄISCHE PATENTANMELDUNG**

(21) Anmeldenummer: **94111574.3**

(22) Anmeldetag: **25.07.94**

(51) Int. Cl.[6]: **C07D 405/00**, C09K 19/34, C09K 19/40, C07F 7/08, C07D 407/00, C07D 491/00, C07D 317/46, C07D 307/78

(30) Priorität: **04.08.93 DE 4326151**

(43) Veröffentlichungstag der Anmeldung: **08.02.95 Patentblatt 95/06**

(84) Benannte Vertragsstaaten: **DE FR GB**

(71) Anmelder: **HOECHST AKTIENGESELLSCHAFT**
**Brüningstrasse 50**
**D-65929 Frankfurt am Main (DE)**

(72) Erfinder: **Fenkl, Franz**
**Schillerstrasse 2**
**D-65451 Kelsterbach (DE)**
Erfinder: **Manero, Dr. Javier**
**Sindlinger Bahnstrasse 163**
**D-65931 Frankfurt (DE)**
Erfinder: **Schlosser, Dr. Hubert**
**Im Hain 2**
**D-61479 Glashütten (DE)**
Erfinder: **Jungbauer, Dr. Dietmar**
**Thomas-Mann-Strasse 8**
**D-64331 Wieterstadt (DE)**

(54) **Aromatische Verbindungen und ihre Verwendung in flüssigkristallinen Mischungen.**

(57) Aromatische Verbindung der Formel (I),

$R^1(-A^1)_a(-M^1)_b(-A^2)_c(-M^2)_d-B(-M^3)_e(-A^3)_f(-M^4)_g(-A^4)_h-R^2$ (I)

in der die Symbole und Indizes folgende Bedeutungen haben:

B ist eine der beiden folgenden aromatischen Gruppen:

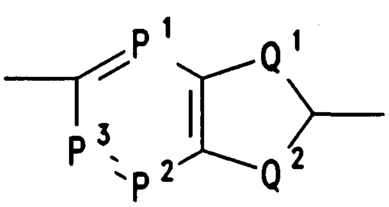

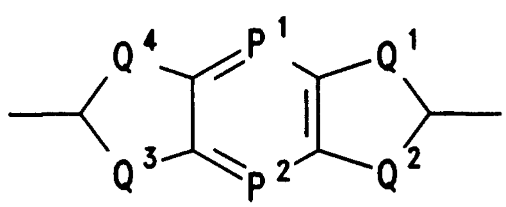

$R^1$, $R^2$ sind gleich oder verschieden Wasserstoff, - CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 20 C-Atomen (mit oder ohne asymmetrisches C-Atom), wobei auch eine oder mehrere $CH_2$-Gruppen durch -O-, -S-, -CO-, -CS-, -CH=CH-, - C≡C-, △, $-Si(CH_3)_2-$, 1,4-Phenylen, trans-1,4-Cyclohexylen oder trans-1,3-Cyclopentylen ersetzt sein können, mit der Maßgabe, daß Sauerstoffatome und Schwefelatome nicht unmittelbar miteinander gebunden sein dürfen, und/oder ein oder mehrere H-Atome des Alkylrestes durch -F, -Cl, -Br, $-OR^2$, - SCN, -OCN oder $-N_3$ substituiert sein können, oder auch eine der nachfolgenden chiralen Gruppen:

EP 0 637 585 A1

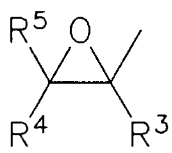
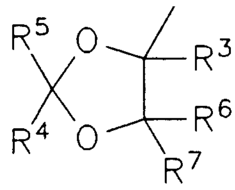
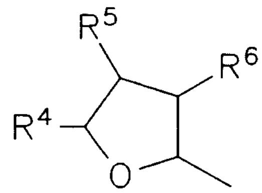

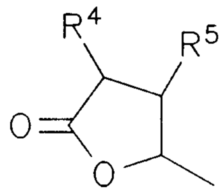
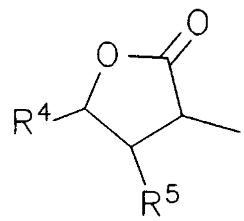
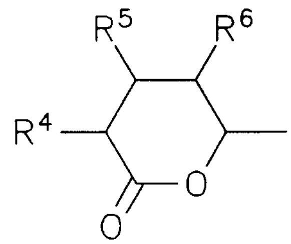

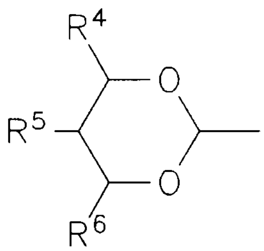
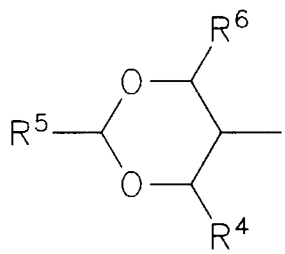
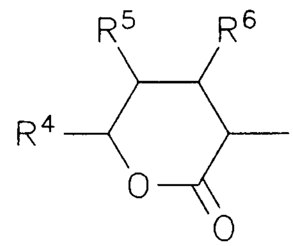

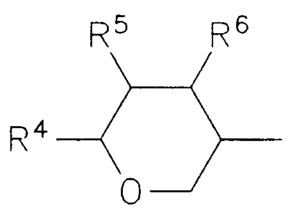
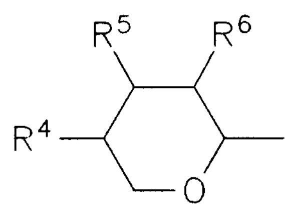
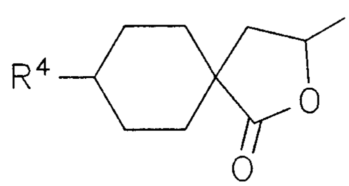

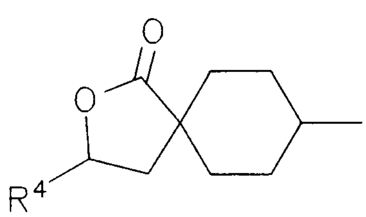
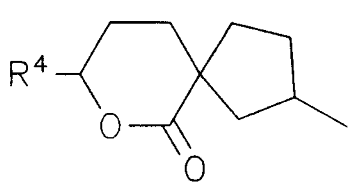
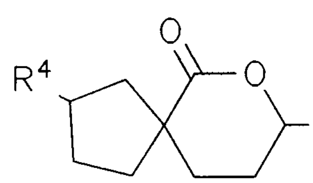

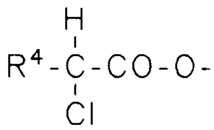 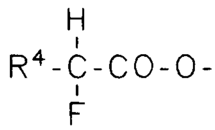 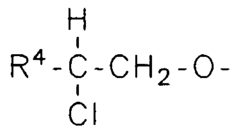 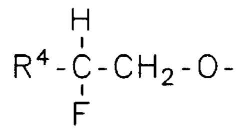

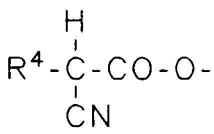 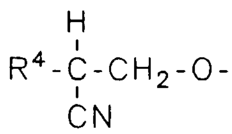 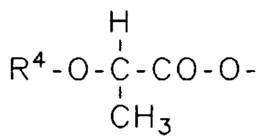 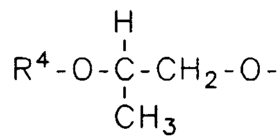

$R^3$, $R^4$, $R^5$, $R^6$, $R^7$ sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- oder -CH = CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4-$ oder $-(CH_2)_5$-sein, wenn sie an ein Oxiran-, Dioxolan-, Tetrahydrofuran-, Tetrahydropyran-, Butyrolacton- oder Valerolacton-System gebunden sind;

$Q^1$, $Q^2$, $Q^3$, $Q^4$ sind gleich oder verschieden $CH_2$, $CF_2$ oder N-$R^3$, S, O,C = O;

$P^1$, $P^2$, $P^3$ sind gleich oder verschieden C-H, C-F oder N;

$M^1$, $M^2$, $M^3$, $M^4$ sind gleich oder verschieden -O-, -S-, -CO-, -CO-O-, -O-CO-, -O-CO-O-, -CO-S-, -S-CO-, -CS-O-, -O-CS-, -S-CS-S-, -O-CS-O, -S-CO-S-, -CS-, $-CH_2-O-$, $-O-CH_2-$, $-CH_2-S-$, $-S-CH_2-$, -CH = CH-, -C≡C-, -O-CO-C≡C-, -C≡C-COO-, -O-CO-CH = CH-, -CH = CH-COO-, $-O-CO-CH_2-CH_2-$, $-CH_2-CH_2-COO-$, $-O-CH_2-$C≡C-, -C≡C-$CH_2$-O-, $-O-CH_2-$CH = CH-, -CH = CH-$CH_2$-O-, $-O-CH_2-CH_2-CH_2-$, $-CH_2-CH_2-CH_2-O-$ oder eine Einfachbindung;

$A^1$, $A^2$, $A^3$, $A^4$, sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Piperazin-1,4-diyl, Piperazin-2,5-diyl, Naphthalin-2,6-diyl, wobei ein oder mehrere H-Atome durch F, Cl und/oder CN ersetzt sein können, Bicyclo[2.2.2]octan-1,4-diyl, wobei ein oder mehrere H-Atome durch F, Cl und/oder CN ersetzt sein können, 1,3-Dioxaborinan-2,5-diyl oder die Gruppe B;

a, b, c, d, e, f, g, h sind null oder eins;

mit der folgenden Maßgabe:
wenn die Gruppe B

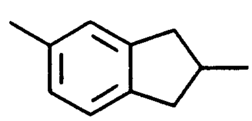

ist, muß mindestens einer der Reste $R^1$ eine $-Si(CH_3)_2-$ und/oder △ Gruppe enthalten und/oder eine der folgenden chiralen Gruppen sein:

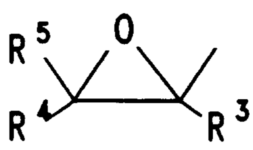

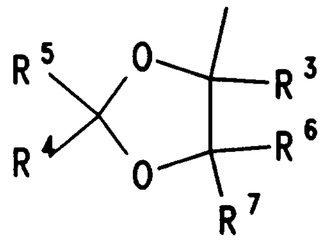

sind geeignet als Komponenten flüssigkristallinen Mischungen.

Neben nematischen und cholesterischen Flüssigkristallen werden in jüngerer Zeit auch optisch aktive geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt-oder Anzeigeelementen führt, die im Vergleich zu den herkömmlichen TN ("twisted nematic")-Zellen um bis zu einem Faktor 1000 schnellere Schaltzeiten haben (siehe z. B. EP-A 0 032 362). Aufgrund dieser und anderer günstiger Eigenschaften, z. B. der bistabilen Schaltmöglichkeit und des nahezu blickwinkelunabhängigen Kontrasts, sind FLCs grundsätzlich für Anwendungsgebiete wie Computerdisplays gut geeignet.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A$ und $S^*_C$-Phase läßt sich z. B. erreichen, wenn die Phasenfolge der Flüssigkristallmischung mit abnehmender Temperatur lautet:

Isotrop $\rightarrow$ $N^*$ $\rightarrow$ $S_A$ $\rightarrow$ $S^*_C$

Vorraussetzung ist, daß der Pitch (Ganghöhe der Helix) in der $N^*$-Phase sehr groß (größer 10 $\mu$m) oder, noch besser, völlig kompensiert ist (siehe z. B. T. Matsumoto et al., p. 468-470, Proc. of the 6th Int. Display Research Conf., Japan Display, Sept. 30 - Okto. 2, 1986, Tokyo, Japan; M. Murakami et al., ibid. S. 344 - S. 347). Dies erreicht man, z. B. indem man zu der chiralen Flüssigkristallmischung, die in der $N^*$-Phase z. B. eine linksdrehende Helix aufweist, einen oder mehrere optisch aktive Dotierstoffe, die eine rechtsdrehende Helix induzieren, in solchen Mengen hinzugibt, daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Vorraussetzung, daß der Pitch in der smektischen C* Phase wesentlich größer ist als die Dicke des Anzeigeelementes (Mol. Cryst. Liq. Cryst. 94 (1983) 213-134 und 114 (1984) 151-187). Dies erreicht man, wie im Fall des cholesterischen Pitches, durch Verwendung von Dotierstoffen mit entgegengesetztem Drehsinn der Helix.

Die optische Schaltzeit $\tau$ [$\mu$s] ferroelektrischer Flüssigkristallsysteme, die möglichst kurz sein soll, hängt von der Rotationsviskosität des Systems $\gamma$ [mPas], der spontanen Polarisation $P_s$[nC/cm$^2$] und der elektrischen Feldstärke E[V/m] ab nach der Beziehung

$$\tau \sim \frac{\gamma}{P_s \bullet E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$, vorzugsweise $\approx$ 0,13, und eine geringe positive oder vorzugsweise negative dielektrische Anisotropie $\Delta\epsilon$ verlangt (siehe z.B. S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Ferroelektrische Flüssigkristallanzeigen lassen sich auch durch Nutzung des DHF (Distorted Helix Formation)-Effektes oder des PSFLCD-Effektes (Pitch Stabilized Ferroelectric Liquid Crystal Display, auch SBF = Short pitch Bistable Ferroelektric Effekt genannt) betreiben. Der DHF-Effekt wurde von B.I. Ostrovski

in Advances in Liquid Crystal Research and Applications, Oxford/Budapest 1980, 469 ff. beschrieben, der PSFLCD-Effekt ist in DE-A 39 20 625 bzw. EP-A 0 405 346 beschrieben. Zur Nutzung dieser Effekte wird im Gegensatz zum SSFLCD-Effekt ein flüssigkristallines Material mit einem kurzen $S_C$-Pitch benötigt.

Da die Entwicklung, insbesondere von ferroelektrischen Flüssigkristallmischungen, noch in keiner Weise als abgeschlossen betrachtet werden kann, sind die Hersteller von Displays an den unterschiedlichsten Komponenten für Mischungen interessiert. Dieses u.a. auch deshalb, weil erst das Zusammenwirken der flüssigkristallinen Mischungen mit den einzelnen Bauteilen der Anzeigevorrichtung bzw. der Zellen (z. B. der Orientierungsschicht) Rückschlüsse auf die Qualität auch der flüssigkristallinen Mischungen zuläßt.

Aufgabe der vorliegenden Erfindung war es daher, neue Verbindungen bereitzustellen, die in flüssigkristallinen Mischungen geeignet sind, das Eigenschaftsprofil dieser Mischungen zu verbessern.

In der JP-A-62 195 355 und der EP-A-0 546 338 sind Indanderivate und deren Verwendung in Flüssigkristallmischungen beschrieben.

Es wurde nun überraschend gefunden, daß kondensierte carbo- und heterocyclische Ringsysteme aus einem aromatischen Sechsring und einem oder zwei aliphatischen Fünfringen in besonderer Weise zum Einsatz in Flüssigkristallmischungen geeignet sind.

Gegenstand der Erfindung sind daher Verbindungen der Formel (I)

$$R^1(-A^1)_a(-M^1)_b(-A^2)_c(-M^2)_d\text{-}B(-M^3)_e(-A^3)_f(-M^4)_g(-A^4)_h\text{-}R^2 \quad \text{(I)}$$

in der die Symbole und Indizes folgende Bedeutungen haben:

B ist eine der beiden folgenden aromatischen Gruppen:

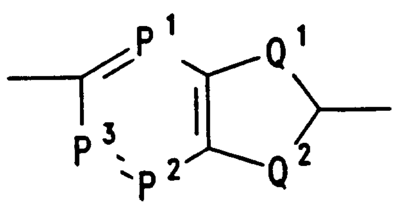

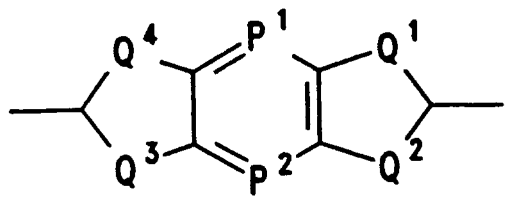

$R^1$, $R^2$ sind gleich oder verschieden Wasserstoff, - CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 20 C-Atomen (mit oder ohne asymmetrisches C-Atom), wobei auch eine oder mehrere $CH_2$-Gruppen durch -O-, -S-,-CO-, -CS-, -CH=CH-, - C≡C-, △, $-Si(CH_3)_2-$, 1,4-Phenylen, trans-1,4-Cyclohexylen oder trans-1,3-Cyclopentylen ersetzt sein können, mit der Maßgabe, daß Sauerstoffatome und Schwefelatome nicht unmittelbar miteinander gebunden sein dürfen, und/oder ein oder mehrere H-Atome des Alkylrestes durch -F, -Cl, -Br, $-OR^2$, - SCN, -OCN oder $-N_3$ substituiert sein können, oder auch eine der nachfolgenden chiralen Gruppen:

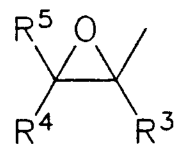

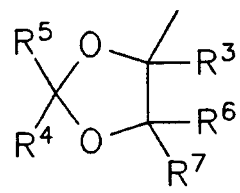

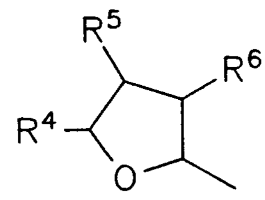

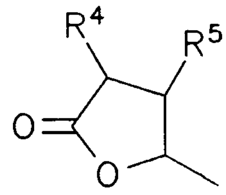

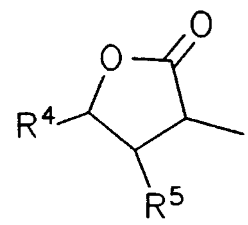

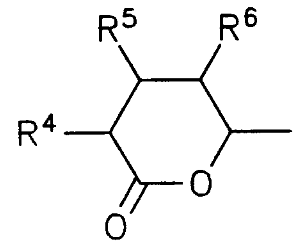

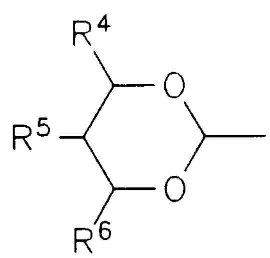

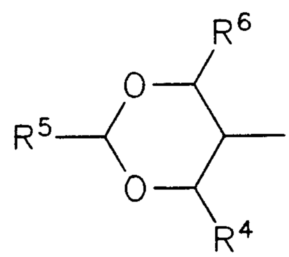

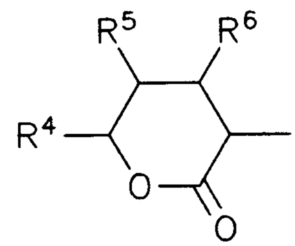

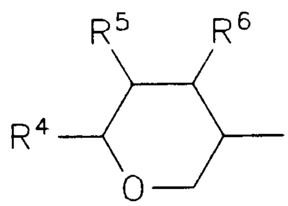

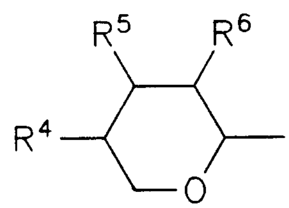

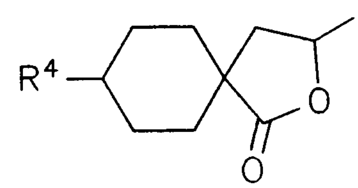

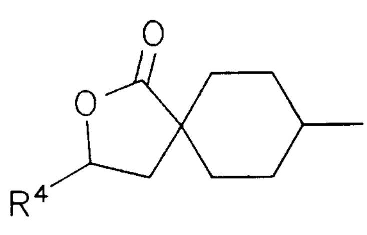

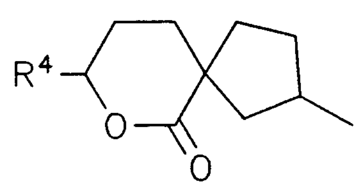

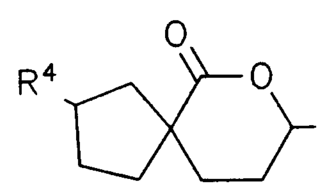

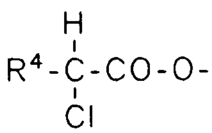 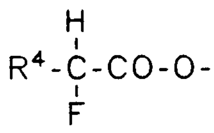 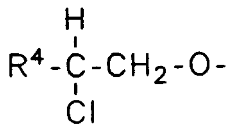 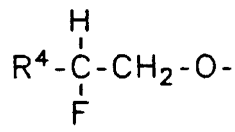

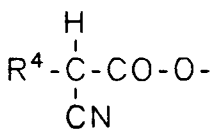 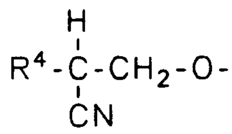 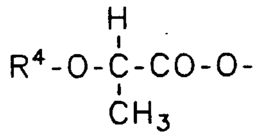 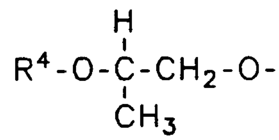

$R^3$, $R^4$, $R^5$, $R^6$, $R^7$ sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- oder -CH=CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4-$ oder $-(CH_2)_5$-sein, wenn sie an ein Oxiran-, Dioxolan-, Tetrahydrofuran-, Tetrahydropyran-, Butyrolacton- oder Valerolacton-System gebunden sind;

$Q^1$, $Q^2$, $Q^3$, $Q^4$ sind gleich oder verschieden $CH_2$, $CF_2$ oder N-$R^3$, S, O,C=O;

$P^1$, $P^2$, $P^3$ sind gleich oder verschieden C-H, C-F oder N;

$M^1$, $M^2$, $M^3$, $M^4$ sind gleich oder verschieden -O-, -S-, -CO-, -CO-O-, -O-CO-, -O-CO-O-, -CO-S-, -S-CO-, -CS-O-, -O-CS-, -S-CS-S-, -O-CS-O, -S-CO-S-, -CS-, $-CH_2-O-$, $-O-CH_2-$, $-CH_2-S-$, $-S-CH_2-$, -CH=CH-, -C≡C-, -O-CO-C≡C-, -C≡C-COO-, -O-CO-CH=CH-, -CH=CH-COO-, $-O-CO-CH_2-CH_2-$, $-CH_2-CH_2-COO-$, $-O-CH_2-C≡C-$, $-C≡C-CH_2-O-$, $-O-CH_2-CH=CH-$, $-CH=CH-CH_2-O-$, $-O-CH_2-CH_2-CH_2-$, $-CH_2-CH_2-CH_2-O-$ oder eine Einfachbindung;

$A^1$, $A^2$, $A^3$, $A^4$, sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Piperazin-1,4-diyl, Piperazin-2,5-diyl, Naphthalin-2,6-diyl, wobei ein oder mehrere H-Atome durch F, Cl und/oder CN ersetzt sein können, Bicyclo[2.2.2]octan-1,4-diyl, wobei ein oder mehrere H-Atome durch F, Cl und/oder CN ersetzt sein können, 1,3-Dioxaborinan-2,5-diyl oder die Gruppe B;

a, b, c, d, e, f, g, h sind null oder eins;

mit der folgenden Maßgabe:
wenn die Gruppe B

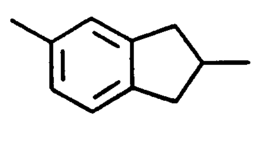

ist, muß mindestens einer der Reste $R^1$ eine $-Si(CH_3)_2-$ und/oder △ Gruppe enthalten und/oder eine der folgenden chiralen Gruppen sein:

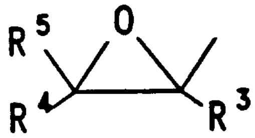

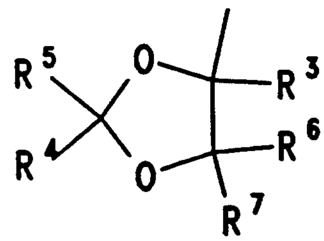

Die erfindungsgemäßen Verbindungen sind unter anderem hervorragend dazu geeignet, in Flüssigkristallmischungen, insbesondere ferroelektrischen, nematische Phasen zu induzieren oder zu verbreitern und ferroelektrische Flüssigkristallmischung mit hohen Werten der Spontanpolarisation $P_s$ herzustellen.

Bevorzugt sind Verbindungen der allgemeinen Formel I, in der die Symbole und Indizes folgende Bedeutungen haben:

$R^1$, $R^2$ sind gleich oder verschieden Wasserstoff, - CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 18 C-Atomen (mit oder ohne asymmetrisches C-Atom), wobei auch eine oder mehrere $CH_2$-Gruppen durch -O-, -CO-, -CH=CH-, -C≡C-, △, $Si(CH_3)_2-$ oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch -F, -Cl, $-OR^3$, -OCN oder $-N_3$ substituiert sein können, oder eine der nachfolgenden chiralen Gruppen:

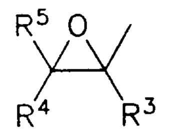 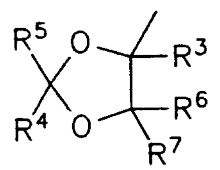 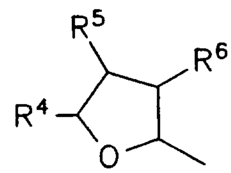

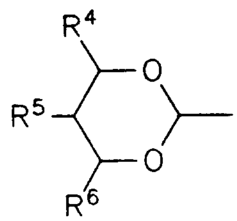 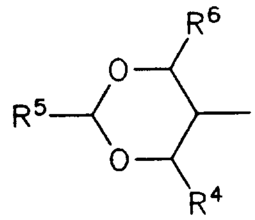 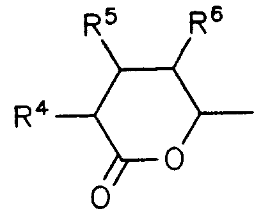

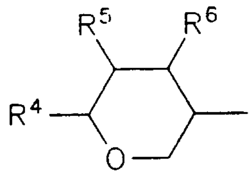 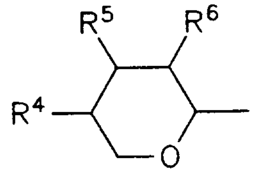 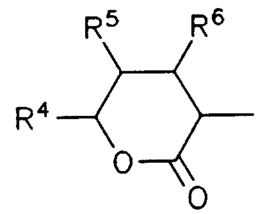

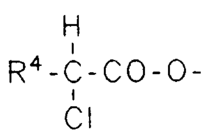 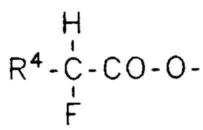 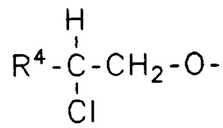 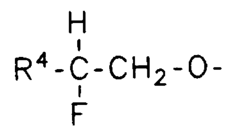

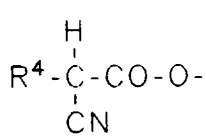 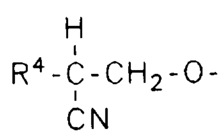 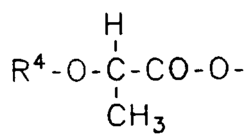 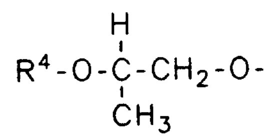

$R^3$, $R^4$, $R^5$, $R^6$, $R^7$ sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- oder -CH = CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4$- oder $-(CH_2)_5$-sein, wenn sie an ein Oxiran-, Dioxolan-, Tetrahydrofuran-, Tetrahydropyran-, Butyrolacton- oder Valerolacton-System gebunden sind;

$Q^1$, $Q^2$, $Q^3$, $Q^4$ sind gleich oder verschieden $CH_2$, $CF_2$ oder N-$R^3$, O, C = O;

$P^1$, $P^2$, $P^3$ sind gleich oder verschieden C-H, C-F oder N;

$M^1$, $M^2$, $M^3$, $M^4$ sind gleich oder verschieden -O-, -CO-, -CO-O-, -O-CO-, -O-CO-O-, -O-CS-O-, $-CH_2$-O-, -O-$CH_2$-, -CH = CH-, -C≡C-, -O-CO-C≡C-, -C≡C-COO-, -O-CO-CH = CH-, -CH = CH-COO-, -O-CO-$CH_2$-$CH_2$-, $-CH_2$-$CH_2$-COO-, -O-$CH_2$-C≡C-, -C≡C-$CH_2$-O-, -O-$CH_2$-CH = CH-, -CH = CH-$CH_2$-O-, -O-$CH_2$-$CH_2$-$CH_2$-, $-CH_2$-$CH_2$-$CH_2$-O- oder eine Einfachbindung;

$A^1$, $A^2$, $A^3$, $A^4$, sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, Thiophen-2,4-diyl, wobei ein H-Atom durch F, Cl und/oder CN

ersetzt sein kann,Thiophen-2,5-diyl, wobei ein oder zwei H-Atome durch F, Cl und/oder CN ersetzt sein können, Naphthalin-2,6-diyl, wobei ein oder mehrere H-Atome durch F, Cl und/oder CN ersetzt sein können, 1,3-Dioxaborinan-2,5-diyl oder die Gruppe B;

a, b, c, d, e, f, g, h sind null oder eins,

wobei die obige Maßangabe gilt.

Ganz besonders bevorzugt sind Verbindungen der allgemeinen Formel I, in der die Symbole und Indizes folgende Bedeutungen haben:

$R^1$, $R^2$ sind gleich oder verschieden Wasserstoff, - CN, -F, -Cl oder ein geradkettiger oder verzweigter Alkylrest mit 1 bis 16 C-Atomen (mit oder ohne asymmetrische C-Atome), wobei auch eine, zwei oder drei $CH_2$-Gruppen durch -O-, -CO-, -CH = CH-, △, $-Si(CH_3)_2$-oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch -F, -Cl oder $-OR^3$ substituiert sein können, oder auch eine der nachfolgenden chiralen Gruppen:

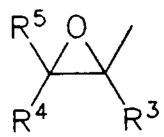
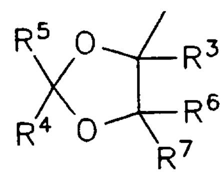
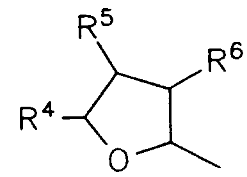

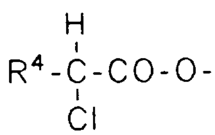
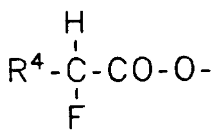
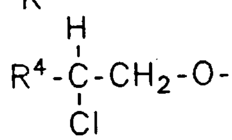
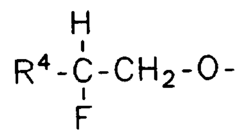

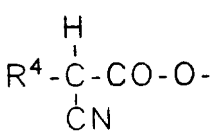
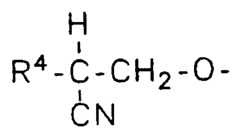
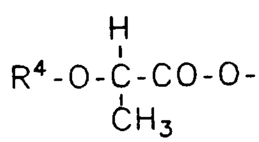
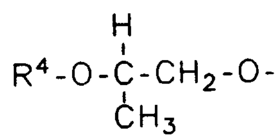

$R^3$, $R^4$, $R^5$, $R^6$, $R^7$ sind gleich oder verschieden Wasserstoff oder ein geradkettiger oder verzweigter Alkylrest mit 1-14 C-Atomen (mit oder ohne asymmetrisches C-Atom), wobei auch eine oder mehrere $CH_2$-Gruppen durch -O- oder -CH = CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4-$ oder $-(CH_2)_5$-sein, wenn sie an ein Oxiran- oder Dioxolan-System gebunden sind;

$Q^1$, $Q^2$, $Q^3$, $Q^4$ sind gleich oder verschieden $CH_2$, $CF_2$ oder N-$R^3$, O, C = O;

$P^1$, $P^2$, $P^3$ sind gleich oder verschieden C-H, C-F oder N;

$M^1$, $M^2$, $M^3$, $M^4$ sind gleich oder verschieden -O-, -CO-, -CO-O-, -O-CO-, -O-CO-O-, $-CH_2$-O-, -O-$CH_2$-, -CH = CH-, - O-CO-C≡C-, -C≡C-COO-, -O-CO-CH = CH-, -CH = CH-COO-, -O-CO-$CH_2$-$CH_2$-,-$CH_2$-$CH_2$-COO-, -O-$CH_2$-C≡C-, -C≡C-$CH_2$-O-, -O-$CH_2$-CH = CH-, -CH = CH-$CH_2$-O-, -O-$CH_2$-$CH_2$-$CH_2$-, -$CH_2$-$CH_2$-$CH_2$-O- oder eine Einfachbindung;

$A^1$, $A^2$, $A^3$, $A^4$ sind gleich oder verschieden 1,4-Phenylen, wobei ein, zwei oder drei H-Atome durch F, Cl und/oder CN ersetzt sein können, Pyrazin-2,5-diyl, wobei ein oder zwei H-Atome durch F, Cl und/oder CN ersetzt sein können,Pyridazin-3,6-diyl, wobei ein oder zwei H-Atome durch F, Cl und/oder CN ersetzt sein können,Pyridin-2,5-diyl, wobei ein oder zwei H-Atome durch F, Cl und/oder CN ersetzt sein können,Pyrimidin-2,5-diyl, wobei ein oder zwei H-Atome durch F, Cl und/oder CN ersetzt sein können,trans-1,4-Cyclohexylen, bei dem ein oder zwei H-Atome durch CN und/oder $CH_3$ ersetzt sein können, (1,3,4)-Thiadiazol-2,5-diyl, Naphthalin-2,6-diyl, wobei ein oder zwei H-Atome durch F, Cl und/oder CN ersetzt

sein können,1,3-Dioxaborinan-2,5-diyl oder die Gruppe B;

a, b, c, d, e, f, g, h sind null oder eins;

mit der Maßgabe, daß mindestens einer der Reste $Q^1$, $Q^2$, $Q^3$, $Q^4$ $-CF_2-$, $-NR^3-$, -S-, -O- oder -CO- ist und/oder mindestens einer der Reste $P^1$, $P^2$, $P^3$ = CF-oder =N- ist.

Ganz besonders bevorzugt sind Verbindungen der allgemeinen Formel I, in der die Gruppe B folgende Bedeutungen hat:

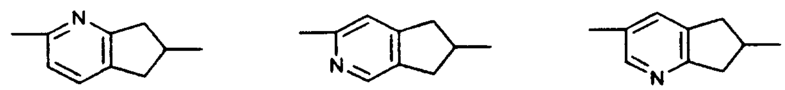
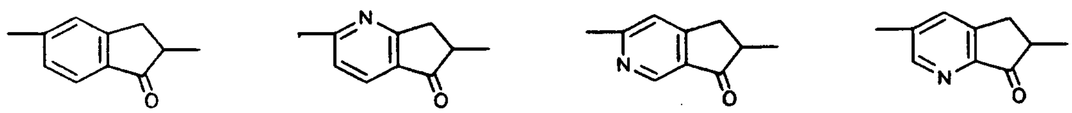
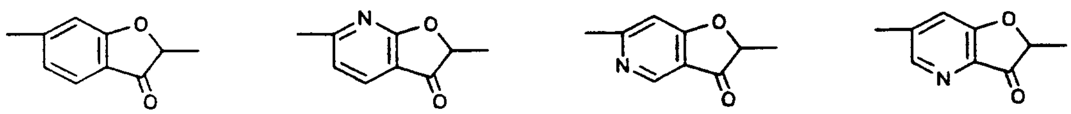
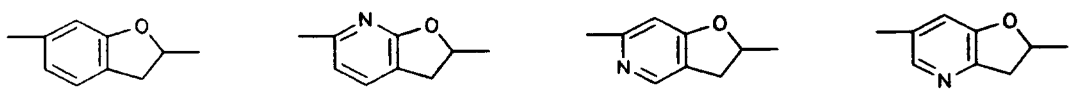
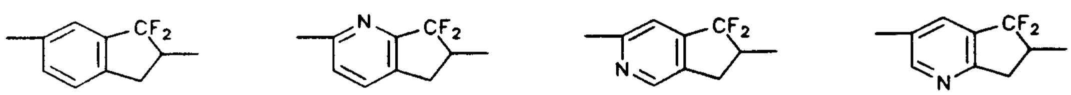
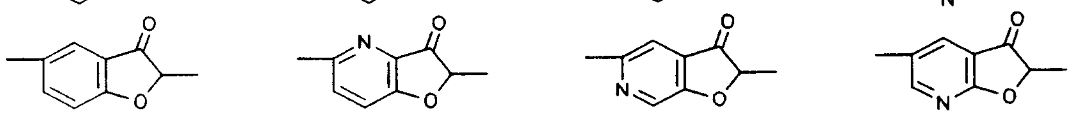
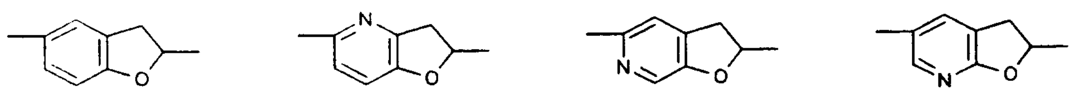
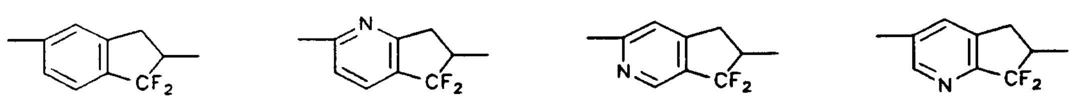
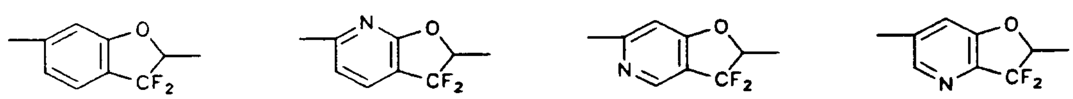
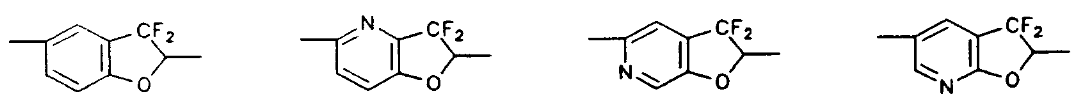
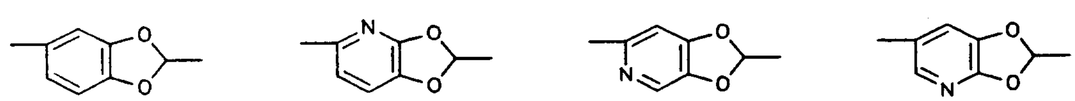
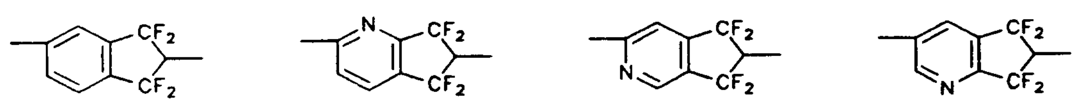

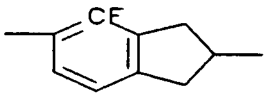
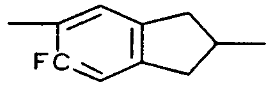
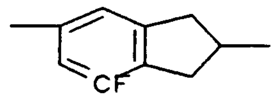
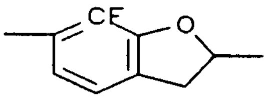
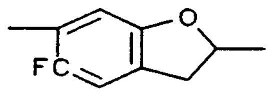
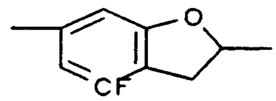
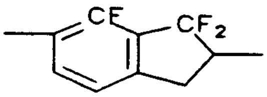
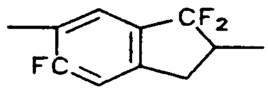
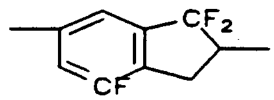
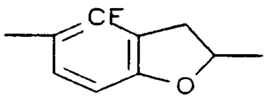
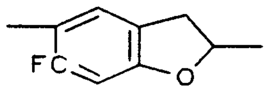
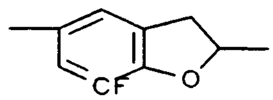
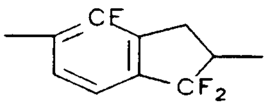
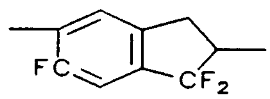
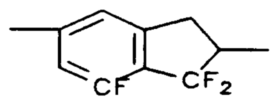
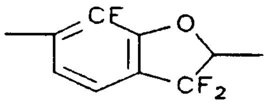
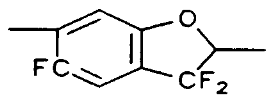
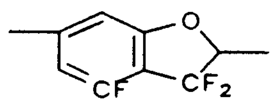
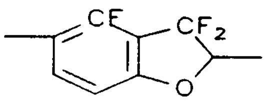
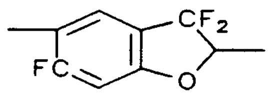
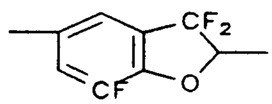
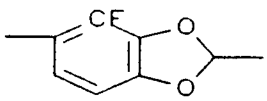
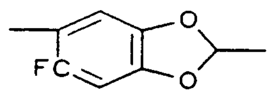
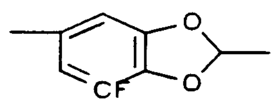
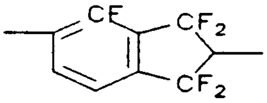
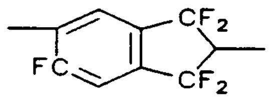
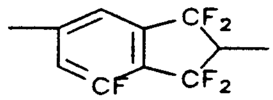
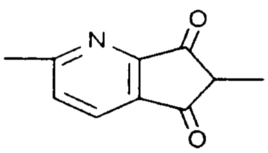
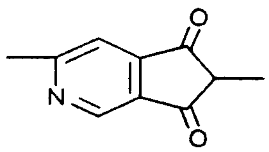
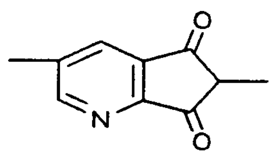
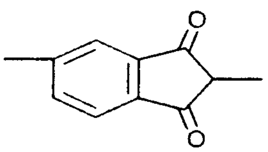

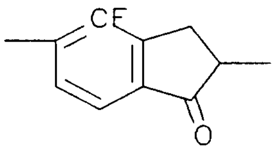 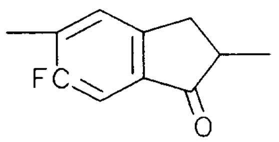 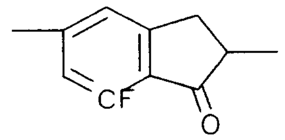

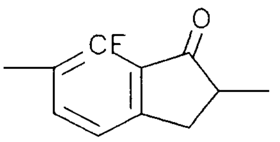 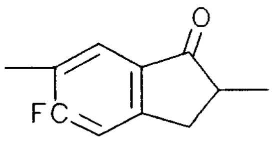 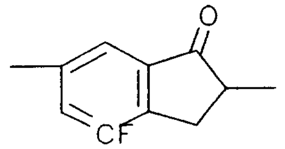

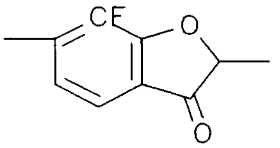 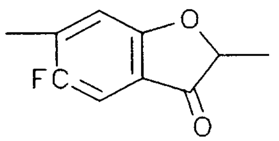 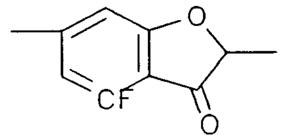

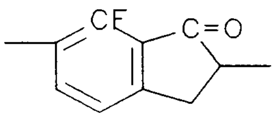 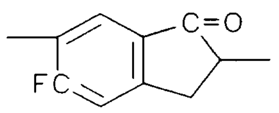 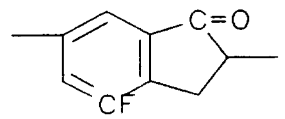

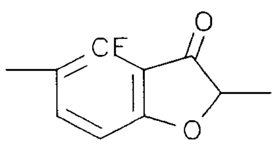 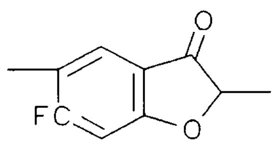 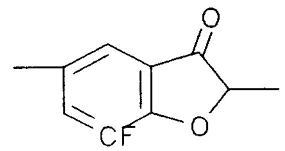

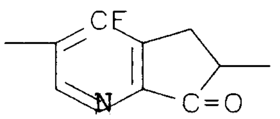 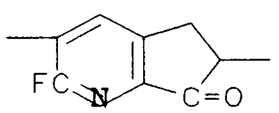 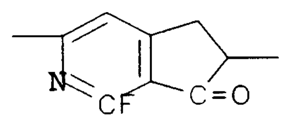

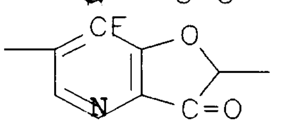 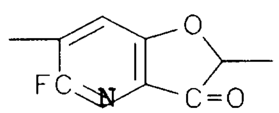 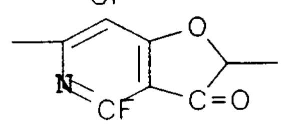

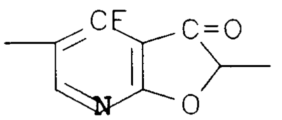 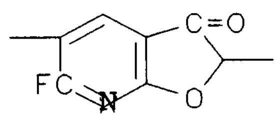 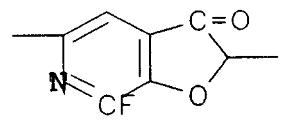

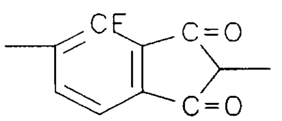 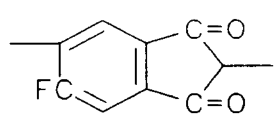 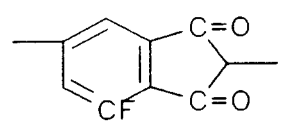

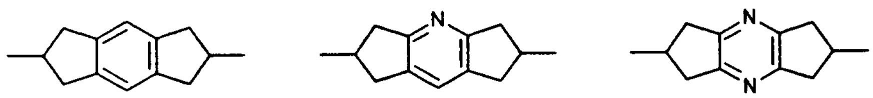

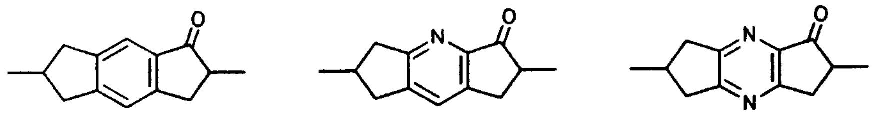

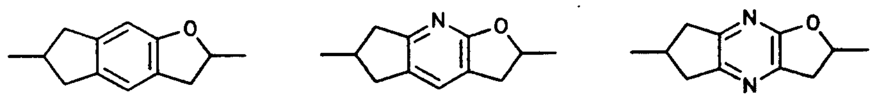

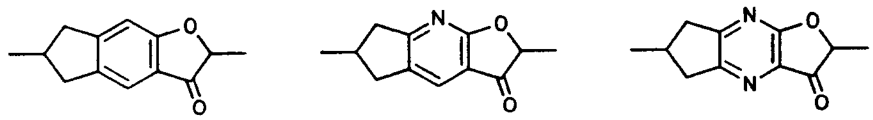

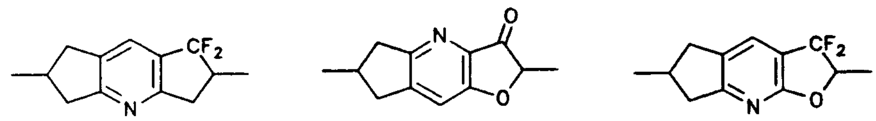

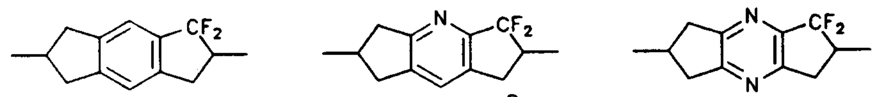

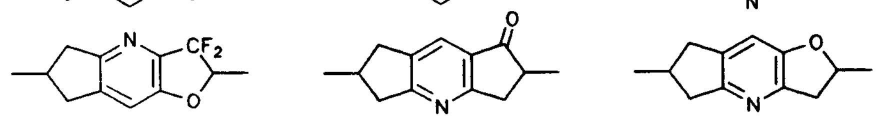

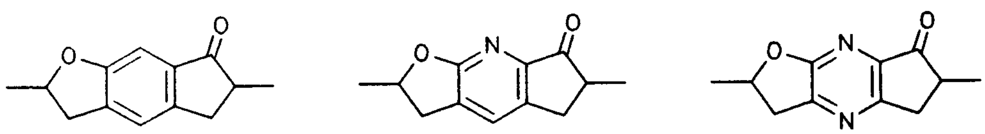

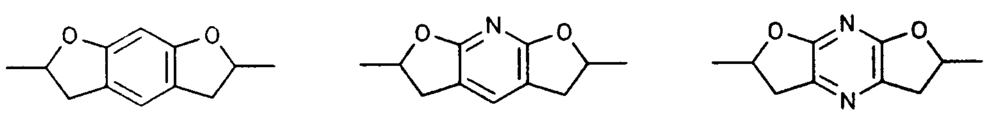

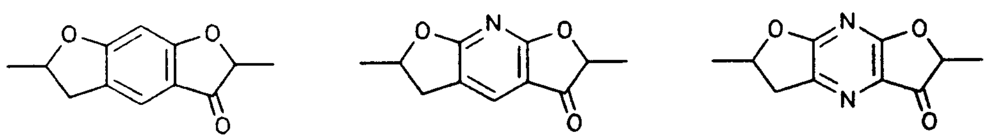

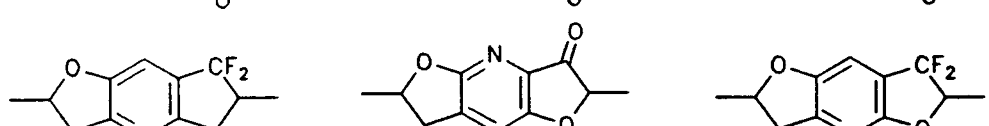

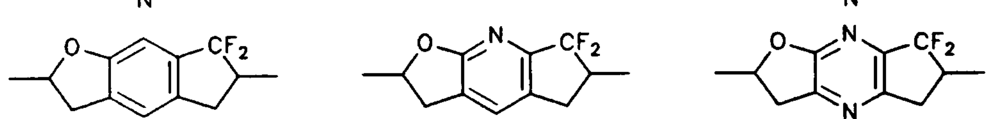

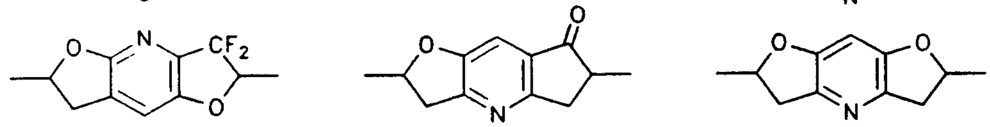

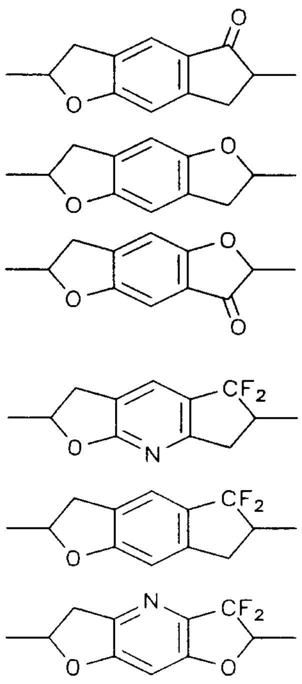
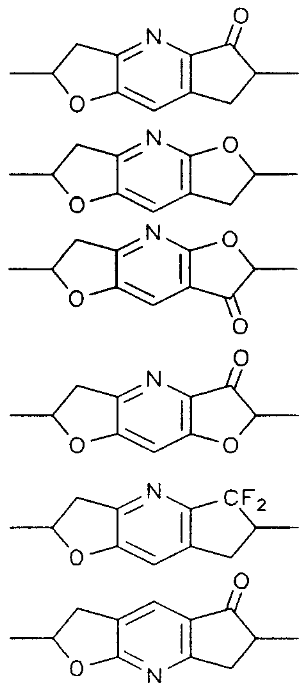
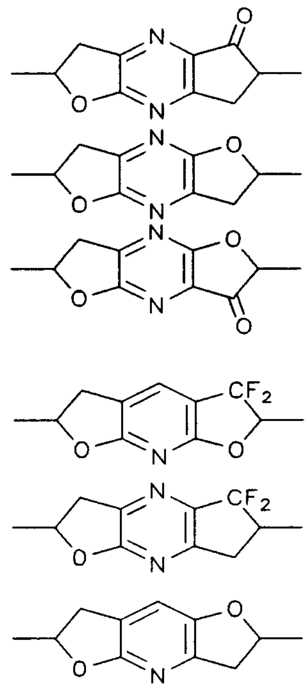

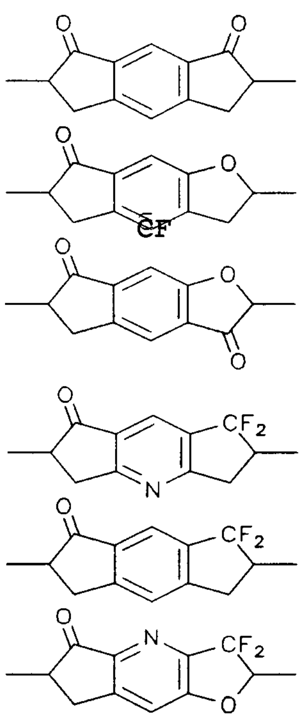
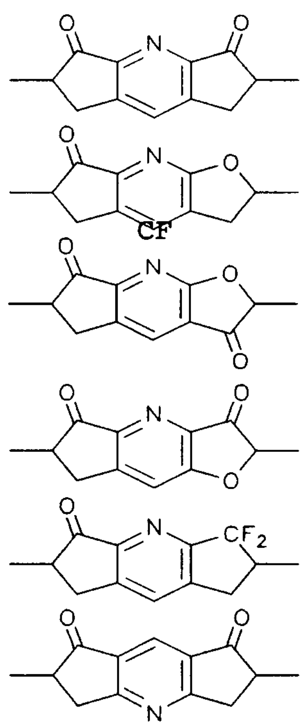
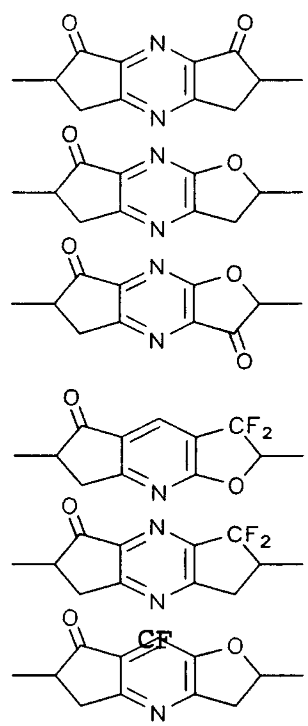

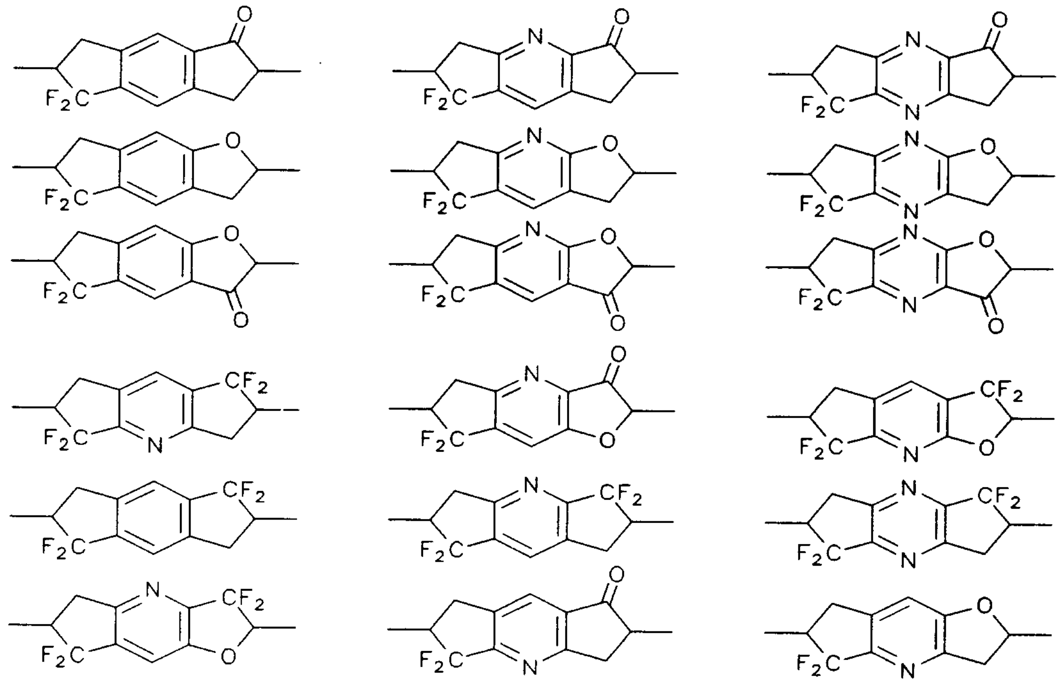

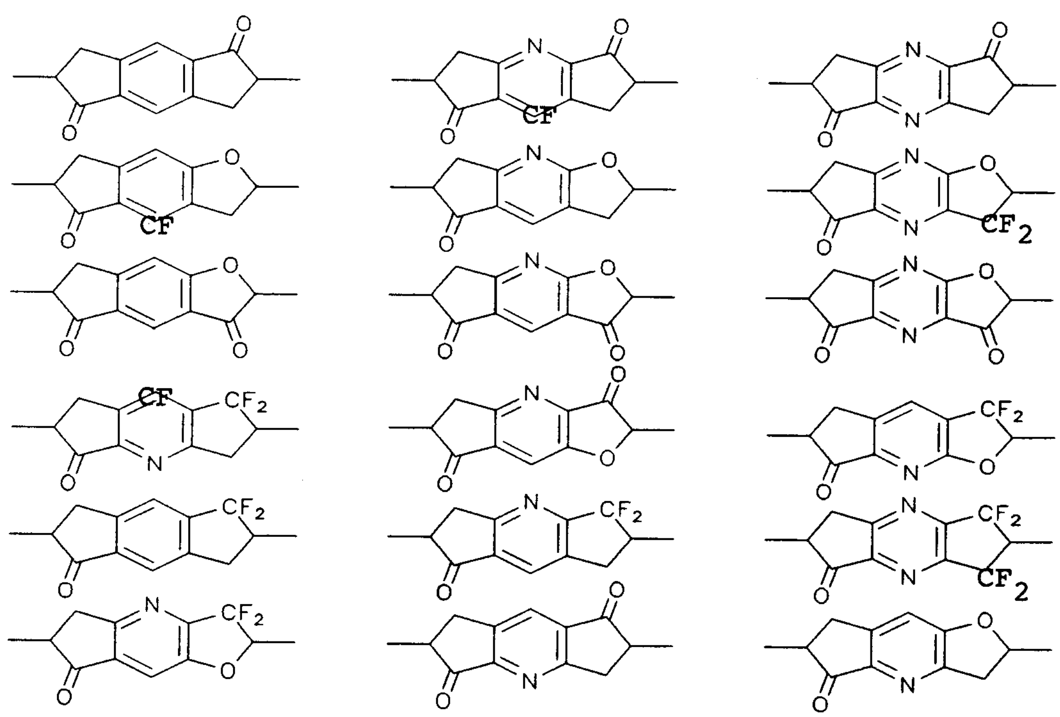

$R^1$, $R^2$ sind gleich oder verschieden Wasserstoff oder ein geradkettiger oder verzweigter Alkylrest mit 1 bis 16 C-Atomen (mit oder ohne asymmetrisches C-Atom), wobei auch eine, zwei oder drei $CH_2$-Gruppen durch -O-, -CO-, -CH = CH-, △, $-Si(CH_3)_2-$ oder trans-1,4-Cyclohexylen ersetzt sein können, mit der Maßgabe, daß Sauerstoffatome nicht unmittelbar miteinander gebunden sein dürfen, und/oder ein oder

mehrere H-Atome des Alkylrestes durch -F oder $-OR^3$ substituiert sein können, oder auch eine der nachfolgenden chiralen Gruppen:

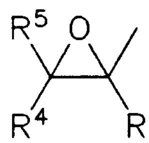

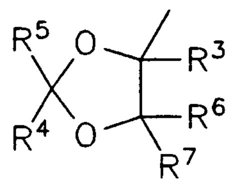

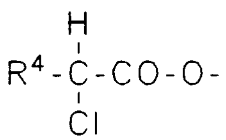

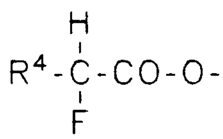

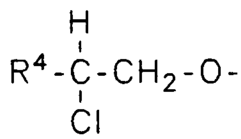

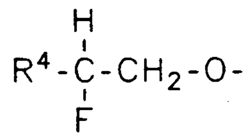

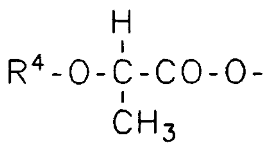

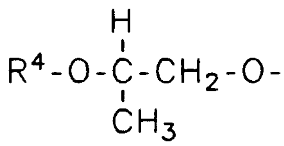

$R^3$, $R^4$, $R^5$, $R^6$, $R^7$ sind gleich oder verschieden Wasserstoff oder ein geradkettiger oder verzweigter Alkylrest mit 1-10 C-Atomen (mit oder ohne asymmetrisches C-Atom), wobei auch eine oder zwei $CH_2$-Gruppen durch -O- oder -CH = CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4-$ oder $-(CH_2)_5$-sein, wenn sie an ein Oxiran- oder Dioxolan-System gebunden sind;

$M^1$, $M^2$, $M^3$, $M^4$ sind gleich oder verschieden -O-, -CO-, -CO-O-, -O-CO-, -O-CO-O-, $-CH_2-O-$, $-O-CH_2-$, -O-CO-C≡C-, -C≡C-COO-, -O-CO-CH = CH-, -CH = CH-COO-, $-O-CO-CH_2-CH_2-$,$-CH_2-CH_2-COO-$, $-O-CH_2-C≡C-$, $-C≡C-CH_2-O-$, $-O-CH_2-CH = CH-$, $-CH = CH-CH_2-O-$, $-O-CH_2-CH_2-CH_2-$, $-CH_2-CH_2-CH_2-O-$ oder eine Einfachbindung;

$A^1$, $A^2$, $A^3$, $A^4$ sind gleich oder verschieden 1,4-Phenylen, wobei ein, zwei oder drei H-Atome durch F, Cl und/oder CN ersetzt sein können, Pyrazin-2,5-diyl, wobei ein oder zwei H-Atome durch F, Cl und/oder CN ersetzt sein können,Pyridazin-3,6-diyl, wobei ein oder zwei H-Atome durch F, Cl und/oder CN ersetzt sein können,Pyridin-2,5-diyl, wobei ein oder zwei H-Atome durch F, Cl und/oder CN ersetzt sein können,Pyrimidin-2,5-diyl, wobei ein oder zwei H-Atome durch F, Cl und/oder CN ersetzt sein können,trans-1,4-Cyclohexylen, bei dem ein oder zwei H-Atome durch CN und/oder $CH_3$ ersetzt sein können, (1,3,4)-Thiadiazol-2,5-diyl oder Naphthalin-2,6-diyl, wobei ein oder zwei H-Atome durch F, Cl und/oder CN ersetzt sein können;

a, b, c, d, e, f, g, h sind null oder eins.

Die Herstellung der erfindungsgemäßen Verbindungen erfolgt nach an sich literaturbekannten Methoden, wie sie in Standardwerken zur Organischen Synthese, z. B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Ein mögliche Synthese von Indan- und Heteroindanderivaten bei denen $Q^1$ gleich $CH_2$ ist , ist in den folgenden Reaktionsschemata A und B dargelegt:

Schema A:

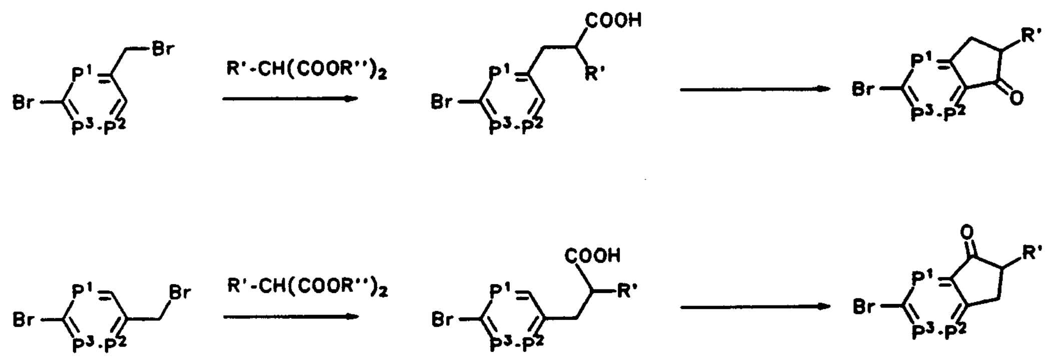


Schema B:

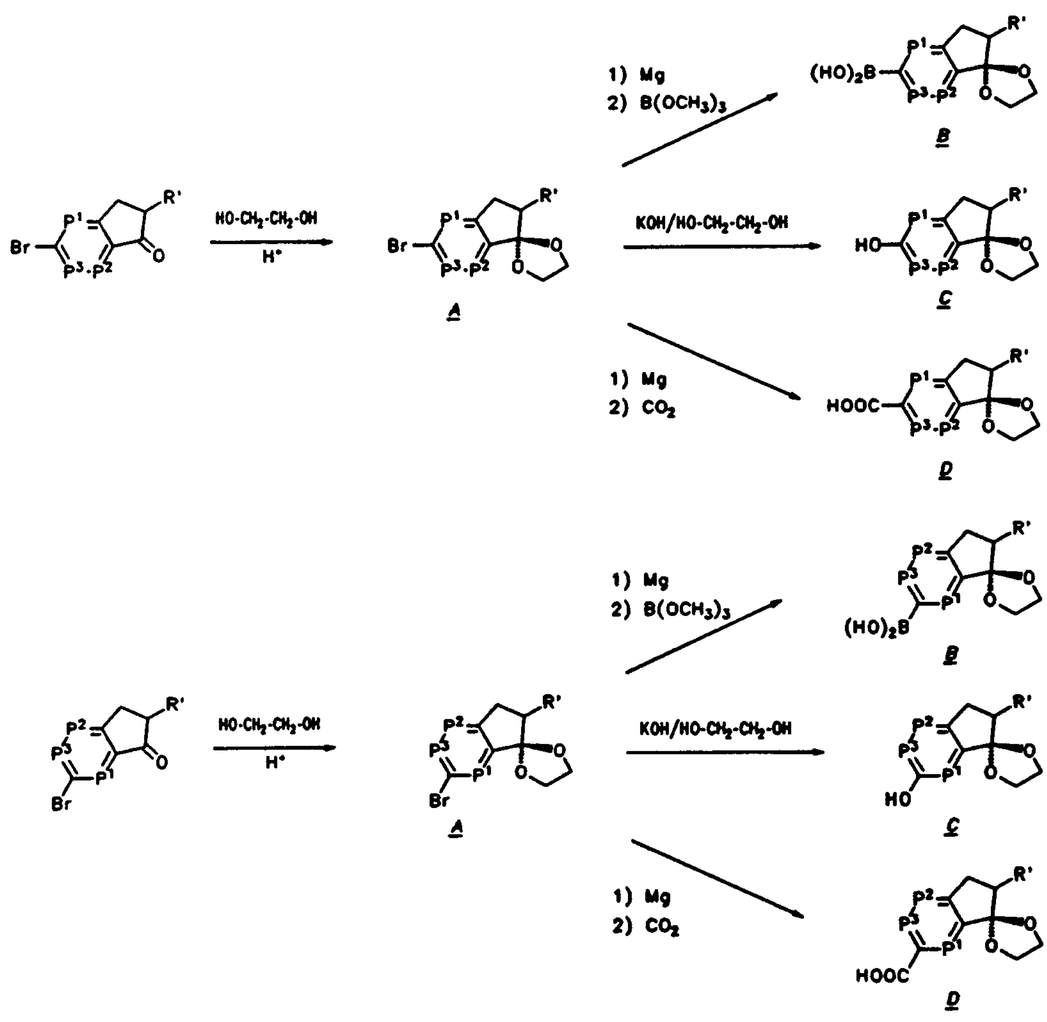


Ein mögliche Synthese von Dihydrobenzofuranen und Heterodihydrobenzofuranderivaten bei denen $Q^1$ gleich O ist , ist in den folgenden Reaktionsschemata C und D dargelegt:

Schema C:

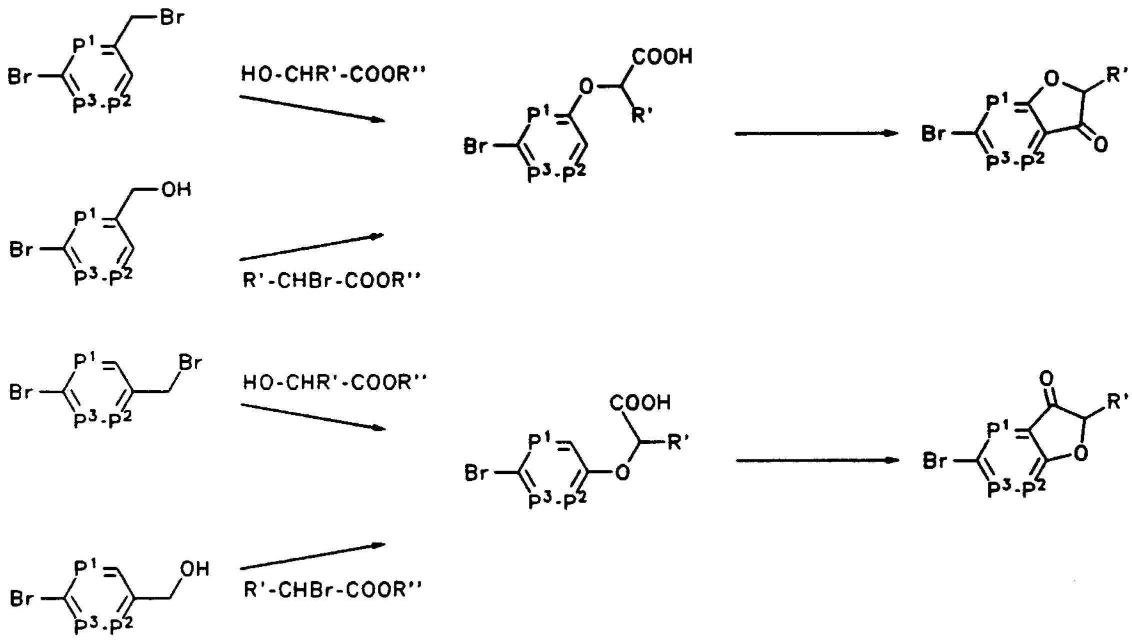

Schema D:

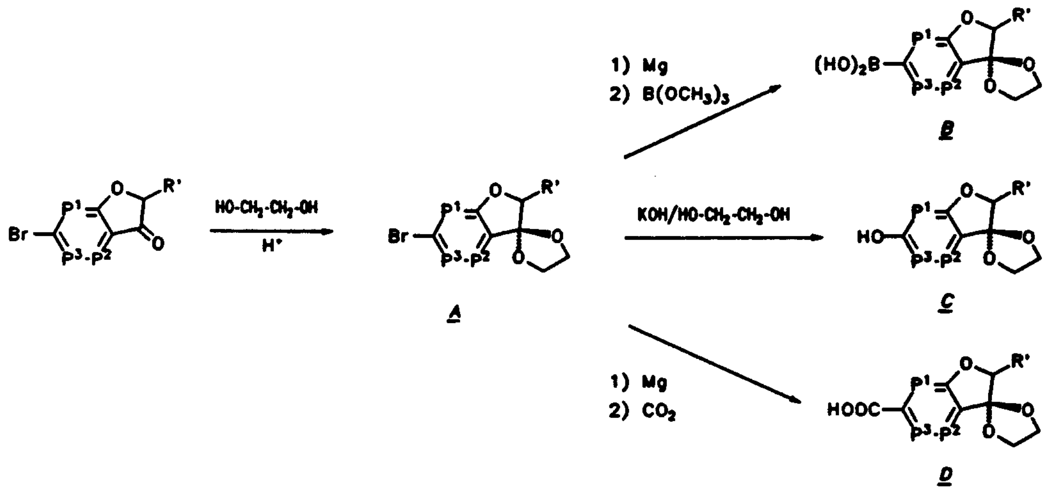


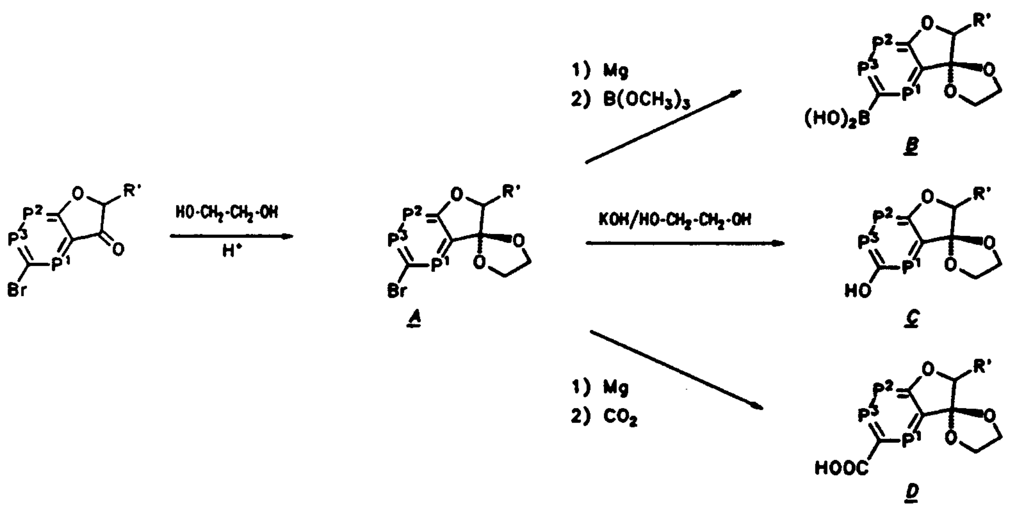


In den folgenden Schemata E und F ist beispielhaft die Synthese von Derivaten gezeigt bei denen $Q^1$ und $Q^2$ gleich O sind:

**Schema E:**

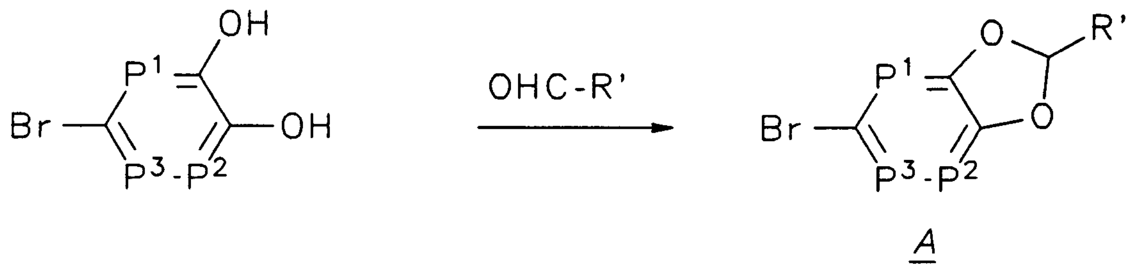


**Schema F**

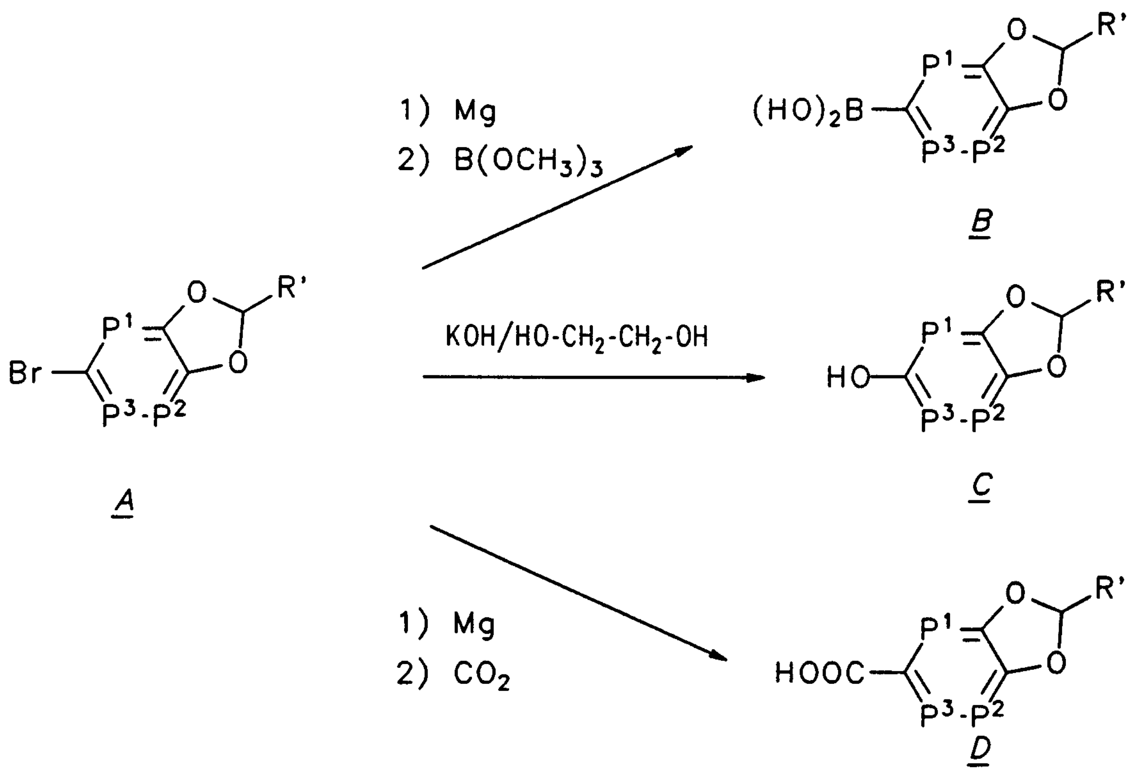


In Schemata A bis F hat R' die Bedeutung $(-M^3)_e(-A^3)_f(-M^4)_g(-A^4)_h-R^x$, wobei $R^x$ gleich $R^2$ oder eine geeignet geschützte Vorstufe für die Ankopplung eines chiralen Fragmentes sein kann. Die Umsetzung der Zwischenverbindungen mit $R^1(-A^1)_a(-M^1)_b(-A^2)_c(-M^2)_d-L$ erfolgt nach gängigen, dem Fachmann an sich bekannten Verfahren. Man kann beispielsweise ***A*** mit Arylboronsäuren unter Übergangsmetallkatalyse in einem inerten Lösungsmittel bei einer Temperatur von -78 °C bis 200 °C kuppeln. Ebenso läßt sich die Boronsäure ***B*** mit geeigneten Arylhalogeniden, wobei in diesem Fall d gleich 0 ist und L Halogen oder $OSO_2-C_kF_{2k+1}$, worin k einen ganzzahligen Wert von 1 bis 10 darstellt, bedeutet, kuppeln. Als Katalysator bei Kupplungen mit Boronsäuren dienen bevorzugt Palladium oder Palladiumverbindungen. Als Lösungsmittel werden beispielsweise Ether, Alkohol, Wasser und Kohlenwasserstoffe oder Gemische verwendet. Weiterhin kann man ***C*** mit Carbonsäuren unter an sich bekannten Bedingungen zu Estern oder mit aliphatischen oder aromatischen Alkoholen oder Phenolen zu Ethern umsetzen. Analog kann man ***D*** nach an sich bekannten Methoden mit aliphatischen oder aromatischen Alkoholen zu Estern umsetzen.

Die Systeme mit zwei aliphatischen Fünfringen am aromatischen Sechsring lassen sich mit, dem Fachmann an sich bekannten Verfahren analog den bisher beschriebenen Methoden herstellen.

Die Synthese des Restes $R^1(-A^1)_a(-M^1)_b(-A^2)_c(-M^2)_d$ bzw. $(-M^3)_e(-A^3)_f(-M^4)_g(-A^4)_hR^2$ erfolgt nach an sich bekannten, dem Fachmann geläufigen Methoden.

Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen.

Beispielsweise sei verwiesen auf DE-A 23 44 732, 24 50 088, 24 29 093, 25 02 904, 26 36 684, 27 01 591 und 27 52 975 für Verbindungen mit 1,4-Cyclohexylen und 1,4-Phenylen-Gruppen; DE-A 26 41 724 für Verbindungen mit Pyrimidin-2,5-diyl-Gruppen; DE-A 40 26 223 und EP-A 03 91 203 für Verbindungen mit Pyridin-2,5-diyl-Gruppen; DE-A 32 31 462 für Verbindungen mit Pyridazin-3,6-diyl-Gruppen; EP-A 309 514 für Verbindungen mit (1,3,4)-Thiadiazol-2,5-diyl-Gruppen; WO-A 92/16500 für Naphthalin-2,6-diyl-Gruppen; DE-A 37 10 890 für Bicyclo[2.2.2.]octan-1,4-diyl-Gruppen; K. Seto et al, Journal of the Chemical Society, Chemical Communications 1988, 56 für Dioxoborinan-2,5-diyl-Gruppen.

Die Herstellung disubstituierter Pyridine, disubstituierter Pyrazine, disubstituierter Pyrimidine und disubstituierter Pyridazine findet sich beispielsweise auch in den entsprechenden Bänden der Serie "The Chemistry of Heterocyclic Compounds" von A. Weissberger und E.C. Taylor (Herausgeber).

Dioxanderivate werden zweckmäßig durch Reaktion eines entsprechenden Aldehyds (oder eines seiner reaktionsfähigen Derivate) mit einem entsprechenden 1,3-Diol (oder einem seiner reaktionsfähigen Derivate) hergestellt, vorzugsweise in Gegenwart eines inerten Lösungsmittels, wie Benzol oder Toluol, und/oder eines Katalysators, z.B. einer starken Säure, wie Schwefelsäure, Benzol- oder p-Toluolsulfonsäure, bei Temperaturen zwischen etwa 20°C und etwa 150°C, vorzugsweise zwischen 80°C und 120°C. Als reaktionsfähige Derivate der Ausgangsstoffe eignen sich in erster Linie Acetale.

Die genannten Aldehyde und 1,3-Diole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester erhältlich.

Verbindungen, worin ein aromatischer Ring durch mindestens ein F-Atom substituiert ist, können auch aus den entsprechenden Diazoniumsalzen durch Austausch der Diazoniumgruppe gegen ein Fluoratom, z.B. nach den Methoden von Balz und Schiemann, erhalten werden.

Was die Verknüpfung der Ringsysteme miteinander angeht, sei beispielsweise verwiesen auf:
N. Miyaura, T. Yanagai und A. Suzuki in Synthetic Communications 11 (1981), 513-519 DE-C-39 30 663, M.J. Sharp, W. Cheng, V. Snieckus in Tetrahedron Letters 28 (1987) 5093; G.W. Gray in J. Chem. Soc. Perkin Trans II 1989, 2041 und Mol. Cryst. Liq. Cryst. 172 (1989) 165, 204 (1991) 43 und 91; EP-A 0 449 015; WO-A 89/12039; WO-A 89/03821; EP-A 0 354 434 für die direkte Verknüpfung von Aromaten und Heteroaromaten; DE-A 32 01 721 für Verbindungen mit $-CH_2CH_2-$Brückengliedern und Koji Seto et al. in Liquid Crystals 8 (1990) 861-870 für Verbindungen mit $-C{\equiv}C-$Brückengliedern.

Ester der Formel (I) können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt und können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls, wie Natrium oder Kalium, in Betracht.

Die Veresterung wird vorteilhaft in Gegenwart eines inerten Lösungsmittels durchgeführt. Gut geeignet sind insbesondere Ether, wie Diethylether, Di-n-butylether, THF, Dioxan oder Anisol, Ketone, wie Aceton, Butanon oder Cyclohexanon, Amide, wie DMF oder Phosphorsäurehexamethyltriamid, Kohlenwasserstoffe, wie Benzol, Toluol oder Xylol, Halogenkohlenwasserstoffe, wie Tetrachlorkohlenstoff, Dichlormethan oder Tetrachlorethylen und Sulfoxide, wie Dimethylsulfoxid oder Sulfolan.

Ether der Formel (I) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, Sulfonat oder Dialkylsulfat umgesetzt werden, zweckmäßig in einem inerten Lösungsmittel, wie Aceton, 1,2-Dimethoxyethan, DMF oder Dimethylsulfoxid, oder auch mit einem Überschuß an wäßriger oder wäßrig-alkoholischer NaOH oder KOH bei Temperaturen zwischen etwa 20° und 100°C.

Was die Synthese spezieller Reste $R^1$, $R^2$ angeht, sei zusätzlich beispielsweise verwiesen auf EP-A 0 355 008 für Verbindungen mit siliziumhaltigen Seitenketten und EP-A 0 292 954 und EP-A 0 398 155 für Verbindungen mit Cyclopropylgruppen in der Seitenkette.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

In diesem Zusammenhang besitzen die Verbindungen der Formel (I) einen breiten Anwendungsbereich. In Abhängigkeit von der Auswahl der Substituenten können sie als Basismaterialien dienen, aus denen flüssigkristalline Phasen zum überwiegenden Teil zusammengesetzt sind; es können aber auch Verbindungen der Formel (I) flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Gegenstand der Erfindung ist auch die Verwendung von Verbindungen der Formel (I) in Flüssigkristallmischungen, vorzugsweise ferroelektrischen, nematischen, antiferroelektrischen und ferrielektrischen, besonders bevorzugt ferroelektrischen und nematischen, insbesondere ferroelektrischen.

Weiterhin Gegenstand der Erfindung sind Flüssigkristallmischungen, vorzugsweise ferroelektrische und nematische, insbesondere ferroelektrische, enthaltend eine oder mehrere Verbindungen der Formel (I).

Die erfindungsgemäßen Flüssigkristallmischungen enthalten im allgemeinen 2 bis 35, vorzugsweise 2 bis 25, besonders bevorzugt 2 bis 20 Komponenten.

Sie enthalten im allgemeinen 0,01 bis 80 Gew.-%, vorzugsweise 0,1 bis 60 Gew.-%, besonders bevorzugt 0,1 bis 30 Gew.-%, an einer oder mehreren, vorzugsweise 1 bis 10, besonders bevorzugt 1 bis 5, ganz besonders bevorzugt 1 bis 3, der erfindungsgemäßen Verbindungen der Formel (I).

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. Dazu gehören z. B.:

- Derivate des Phenylpyrimidins, wie beispielsweise in WO 86/06401, US-A 4 874 542 beschrieben,
- metasubstituierte Sechsringaromaten, wie beispielsweise in der deutschen Patentanmeldung P 42 22 565 beschrieben,
- Siliziumverbindungen, wie beispielsweise in EP-A 0 355 008 beschrieben,
- mesogene Verbindungen mit nur einer Seitenkette, wie in EP-A 0 541 081 beschrieben,
- Hydrochinonderivate, wie beispielsweise in der deutschen Patentanmeldung P 4 243 705 beschrieben,
- Pyridylpyrimidine, wie beispielsweise in WO 92/12974 beschrieben,
- Phenylbenzoate, wie beispielsweise bei P. Keller, Ferroelectrics 58 (1984), 3 und J. W. Goodby et al., Liquid Crystals and Ordered Fluids, Bd. 4, New York 1984 beschrieben und
- Thiadiazole, wie z. B. in EP-B 309 514 beschrieben.

Als chirale, nicht racemische Dotierstoffe kommen beispielsweise in Frage:

- optisch aktive Phenylbenzoate, wie beispielsweise bei P. Keller, Ferroelectrics 58 (1984), 3 und J. W. Goodby et al., Liquid Crystals and Ordered Fluids, Bd. 4, New York 1984 beschrieben,
- optisch aktive Oxiranether, wie beispielsweise in EP-A 0 263 437 und WO-A 93/13093 beschrieben,
- optisch aktive Oxiranester, wie beispielsweise in EP-A 0 292 954 beschrieben,
- optisch aktive Dioxolanether, wie beispielsweise in EP-A 0 351 746 beschrieben,
- optisch aktive Dioxolanester, wie beispielsweise in EP-A 0 361 272 beschrieben, und
- optisch aktive Tetrahydrofuran-2-carbonsäureester, wie beispielsweise in EP-A 0 355 561 beschrieben.

Insbesondere bevorzugt als weitere Komponenten der erfindungsgemäßen Mischungen sind Verbindungen der Formel II bis XVI:

A. Phenylpyrimidine der Formel (II),

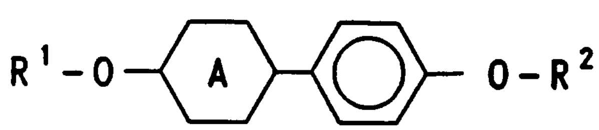

(II)

worin bedeuten:

$R^1$, $R^2$ gleich oder verschieden, eine geradkettige oder verzweigte Alkylgruppe mit 1 bis 18 bzw. 3

bis 18 C-Atomen, wobei in einem oder beiden der Reste $R^1$, $R^2$ die dem Sauerstoff benachbarte $CH_2$-Gruppe auch durch -CO- ersetzt sein kann,

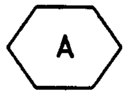 : 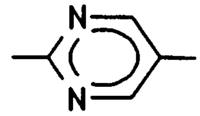 oder 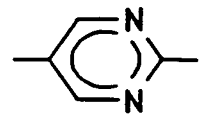 ,

und (III),

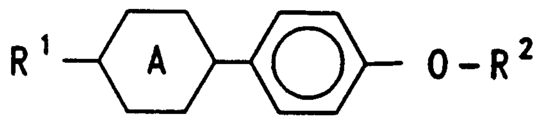 (III)

worin bedeuten:

$R^1$, $R^2$ gleich oder verschieden, eine verzweigte oder unverzweigte Alkylgruppe mit 1 bis 18 bzw. 3 bis 18 C-Atomen, wobei eine dem Sauerstoff benachbarte $CH_2$-Gruppe auch durch -CO- ersetzt sein kann;

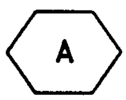 : 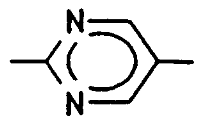 oder 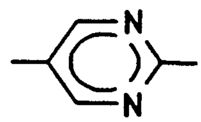 .

B. metasubstituierte Sechsringaromaten der Formel (IV),

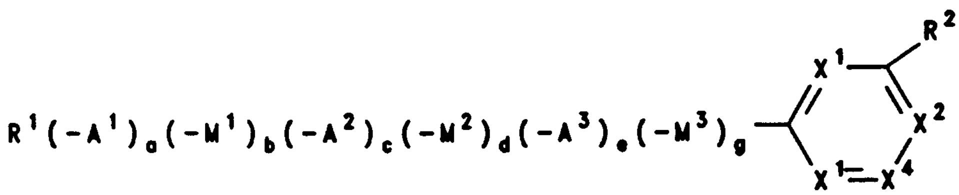 (IV)

worin bedeuten:

| | |
|---|---|
| $R^1$, $R^2$ | gleich oder verschieden einen geradkettigen oder verzweigten Alkylrest mit 1 bis 22 bzw. 3 bis 22 C-Atomen, wobei auch eine oder zwei nicht benachbarte $CH_2$-Gruppen durch -O-, -CO-, -CO-O-, -O-CO-, -O-CO-O- oder $-Si(CH_3)_2$-ersetzt sein können; |
| $A^1$, $A^2$, $A^3$ | sind gleich oder verschieden, 1,4-Phenylen, wobei ein oder zwei H-Atome durch F ersetzt sein können, Pyridin-2,5-diyl, wobei ein, zwei oder drei H-Atome durch F ersetzt sein können, Pyrimidin-2,5-diyl, wobei dem ein oder zwei H-Atome durch F ersetzt sein können, trans-1,4-Cyclohexylen, bei dem ein oder zwei H-Atome durch -CN und/oder $-CH_3$ ersetzt sein können, (1,3,4)-Thiadiazol-2,5-diyl, und |
| $A^1$ | auch |

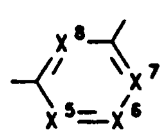

| | |
|---|---|
| $M^1$, $M^2$, $M^3$ | gleich oder verschieden, -O-, -CO-O-, - O-CO-, $-CH_2-O-$, $-O-CH_2-$, $-CH_2-CH_2-$; |
| $X^1$, $X^2$, $X^3$, $X^4$, $X^5$, $X^5$, $X^7$, $X^5$ | gleich oder verschieden =CH-, =CF- oder =N-, wobei die Zahl der |

| | |
|---|---|
| | N-Atome je Sechsring 0,1 oder 2 beträgt, |
| a, b, c, d, e, f | sind null oder eins, unter der Bedingung, daß die Summe aus a + c + e 0, 1 oder 2 ist. |

C. Carbonate der Formel (V),

$R^1$-O-CO-O-(-$A^1$)$_a$(-$M^1$)$_b$(-$A^2$)$_c$(-$M^2$)$_d$(-$A^3$)$_e$(-O)$_f$-$R^2$ (V)

worin bedeuten;

| | |
|---|---|
| $R^1$, $R^2$ | gleich oder verschieden eine geradkettige oder verzweigte Alkylgruppe mit 1 bis 22 bzw. 3 bis 22 C-Atomen; |
| $A^1$, $A^2$, $A^3$, | gleich oder verschieden, 1,4-Phenylen, wobei auch ein, zwei oder drei H-Atome durch F ersetzt sein können, Pyrimidin-2,5-diyl, wobei auch ein oder zwei H-Atome durch F ersetzt sein können, Pyridin-2,5-diyl, wobei auch ein oder zwei H-Atome durch F ersetzt sein können; |
| $M^1$, $M^2$, | gleich oder verschieden, -O-, -CO-, -CO-O-, -O-CO-, -$CH_2$-O-, -O-$CH_2$- oder -$CH_2$-$CH_2$- ; |
| a, b, c, d, e , f | null oder eins, unter der Bedingung, daß die Summe a + c + e 1, 2 oder 3 ist. |

D. Siliziumverbindungen der Formel (VI),

$R^1$(-$A^1$)$_i$(-$M^1$)$_k$(-$A^2$)$_l$(-$M^2$)$_m$(-$A^3$)$_n$-$R^2$ (VI)

worin bedeuten:

| | |
|---|---|
| $R^1$ | eine geradkettige oder verzweigte Alkylgruppe mit 1 bis 22 bzw. 3 bis 22 C-Atomen, wobei auch eine oder zwei nicht benachbarte $CH_2$-Gruppen durch -O-, -CO-, -CO-O-, -O-CO- oder -O-CO-O-ersetzt sein können; |
| $R^2$ | geradkettiges oder verzweigtes Alkyl mit 1 bis 22 bzw. 3 bis 22 C-Atomen, wobei auch eine oder zwei nicht benachbarte $CH_2$-Gruppen durch -O-, -CO-, -CO-O-, -O-CO- oder -O-CO-O-ersetzt sein können, mit der Maßgabe, daß eine, nicht an Sauerstoff gebundene, $CH_2$-Gruppe durch -Si($CH_3$)$_2$- ersetzt ist; |
| $A^1$, $A^2$, $A^3$ | gleich oder verschieden 1,4-Phenylen, wobei ein, zwei oder drei H-Atome durch F ersetzt sein können, trans-1,4-Cyclohexylen, Pyridin-2,5-diyl, wobei ein oder zwei H-Atome durch F ersetzt sein können, Pyrimidin-2,5-diyl, wobei ein oder zwei H-Atome jeweils durch F ersetzt sein können, (1,3,4)-Thiadiazol-2,5-diyl; |
| $M^1$, $M^2$ | gleich oder verschieden, -CO-O-, -O-CO-, -$CH_2$-O-, -O-$CH_2$-; i, k, l, m, n null oder 1, mit der Maßgabe, daß i + l + n = 2 oder 3 ist. |

E. Hydrochinonderivate der Formel (VII),

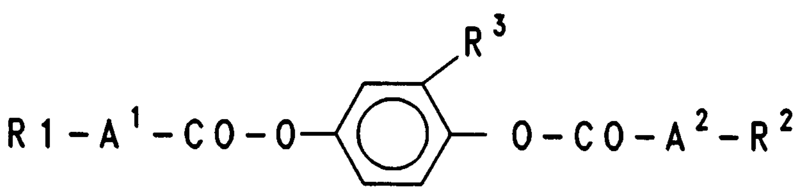 (VII)

worin bedeuten:

| | |
|---|---|
| $R^1$, $R^2$ | gleich oder verschieden einen geradkettigen oder verzweigten Alkylrest mit 1 bzw. 3 bis 16 vorzugsweise 1 bzw. 3 bis 10 C-Atomen, wobei auch eine oder zwei nicht benachbarte $CH_2$-Gruppen durch -O-, -CO-, -O-CO-, -CO-O-, -O-CO-O-, ersetzt sein können; |
| $R^3$ | -$CH_3$, -$CF_3$ oder -$C_2H_5$; |
| $A^1$, $A^2$ | gleich oder verschieden |

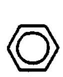 , 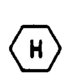 .

F. Pyridylpyrimidine der Formel (VIII),

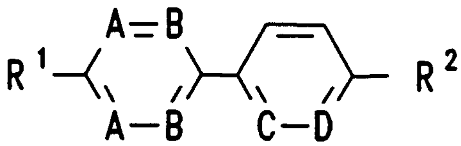 (VIII)

wobei bedeuten:

A gleich N und B gleich CH oder A gleich CH und B gleich N, C gleich N und D gleich CH oder C gleich CH und D gleich N,

wobei eine oder zwei CH-Gruppen durch CF-Gruppen ersezt sein können;

- $R^1$, $R^2$ gleich oder verschieden einen geradkettigen oder verzweigten Alkylrest mit 1 bis 22 bzw. 3 bis 22 C-Atomen, wobei auch eine oder zwei nicht benachbarte $CH_2$-Gruppen durch -O-, -CO-, -CO-O-, -O-CO- oder -O-CO-O- ersetzt sein können.

G. Phenylbenzoate der Formel (IX),

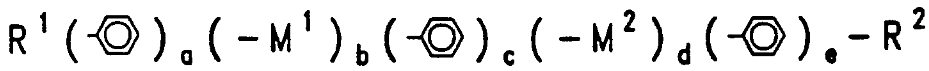 (IX)

wobei bedeuten

- $R^1$, $R^2$ gleich oder verschiedenen einen geradkettigen oder verzweigten Alkylrest mit 1 bis 22 bzw. 3 bis 22 C-Atomen, wobei auch eine oder zwei nicht benachbarte $CH_2$-Gruppen durch -O-, -CO-, -CO-O-, -O-CO- oder -O-CO-O- ersetzt sein können;
- $M^1$, $M^2$ gleich oder verschieden, -CO-O-, -O-CO-;
- a, b, c, d, e null oder eins, unter der Bedingung, daß $a + c + e = 2$ oder 3 und $b + d = 1$ oder 2.

H. Verbindungen mit nur einer Seitenkette der Formel (X),

$R^1(-A^1)_a(-M^1)_b(-A^2)_c(-M^2)_d(-A^3)_e(-M^3)_f(-A^4)-H$ (X)

wobei bedeuten:

- $R^1$ geradkettiger oder verzweigter Alkylrest mit 1 bis 22 bzw. 3 bis 22 C-Atomen, wobei auch eine oder zwei nicht benachbarte $CH_2$-Gruppen durch -O-, -CO-, -CO-O-, -O-CO-, -O-CO-O- oder $-Si(CH_3)_2-$ ersetzt sein können;
- $A^1$, $A^2$, $A^3$, $A^4$ gleich oder verschieden, 1,4-Phenylen, wobei ein, zwei oder drei H-Atome durch F oder CN ersetzt sein können, Pyridin-2,5-diyl, bei dem ein oder zwei H-Atome durch F ersetzt sein können, Pyrimidin-2,5-diyl, bei dem ein oder zwei H-Atome durch F ersetzt sein können, trans-1,4-Cyclohexylen, (1,3,4)-Thiadiazol-2,5-diyl oder Naphthalin-2,6-diyl;
- $M^1$, $M^2$, $M^3$ gleich oder verschieden, -CO-O-, -O-CO-, $-CH_2-O-$, $- O-CH_2-$oder $-CH_2-CH_2-$;
- a, b, c, d, e, f null oder eins, unter der Bedingung, daß die Summe aus $a + c + e$ 0, 1, 2 oder 3 ist.

I. Optisch aktive Phenylbenzoate der Formel (XI),

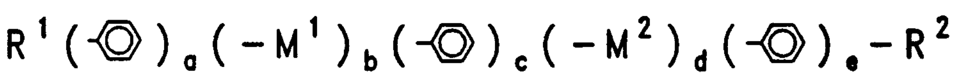 (XI)

wobei bedeuten

- $R^1$, $R^2$ gleich oder verschieden ein geradkettiger oder verzweigter Alkylrest mit 1 bis 22 bzw. 3 bis 22 C-Atomen, wobei auch eine oder zwei nicht benachbarte $CH_2$-Gruppen durch -O-, -CO-, -CO-O-, -O-CO- oder -O-CO-O- ersetzt sein können, und worin wenigstens einer der Reste $R^1$, $R^2$ chiral und nicht racemisch ist;
- $M^1$, $M^2$ gleich oder verschieden, -CO-O-, -O-CO- oder Einfachbindung;
- a, b, c, d, e sind null oder eins, unter der Bedingung, daß $a + c + e$ 2 oder 3 ist.

J. Optisch aktive Oxiranether der Formel (XII),

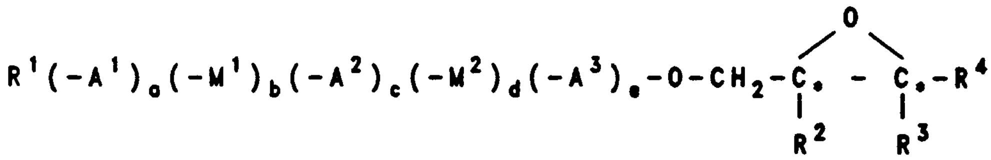

(XII)

wobei die Symbole und Indizes folgende Bedeutung haben:

| | |
|---|---|
| * | chirales Zentrum; |
| $R^1$ | ein geradkettiger oder verzweigter Alkylrest mit 1 bis 22 bzw. 3 bis 22 C-Atomen, wobei auch eine oder zwei nicht benachbarte $CH_2$-Gruppen durch -O-, -CO-, -CO-O-, -O-CO-, -O-CO-O- oder $-Si(CH_3)_2$-ersetzt sein können oder die nachfolgende, optisch aktive Gruppe: 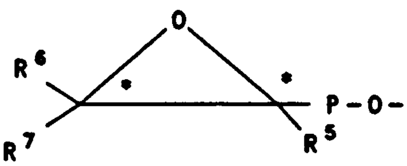 |
| $R^2$,$R^3$,$R^4$,$R^5$,$R^6$,$R^7$ | gleich oder verschieden, H oder ein geradkettiger oder verzweigter Alkylrest mit 1 bis 16 bzw. 3 bis 16 C-Atomen; |
| P | $-CH_2-$ oder -CO- ; |
| $A^1$, $A^2$, $A^3$ | gleich oder verschieden, 1,4-Phenylen wobei ein, zwei oder drei H-Atome durch F ersetzt sein können, Pyridin-2,5-diyl, wobei ein oder zwei H-Atome jeweils durch F ersetzt sein können, Pyrimidin-2,5-diyl, wobei ein oder zwei H-Atome durch F ersetzt sein können, trans-1,4-Cyclohexylen, bei dem ein oder zwei H-Atome durch -CN und/oder $-CH_3$ ersetzt sein können, (1,3,4)-Thiadiazol-2,5-diyl; |
| $M^1$, $M^2$ | gleich oder verschieden, -CO-O-, -O-CO-, $-CH_2-O-$, $-O-CH_2-$, $-CH_2-CH_2-$; |
| a, b, c, d, e | null oder eins. |

K. Optisch aktive Oxiranester der Formel (XIII),

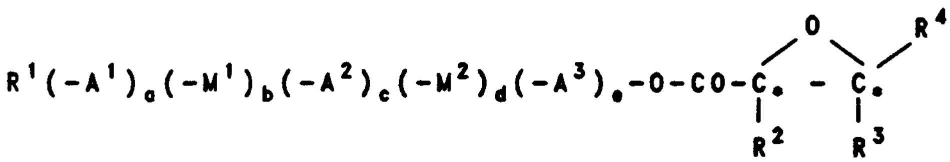

(XIII)

wobei die Symbole und Indizes folgende Bedeutung haben:

| | |
|---|---|
| * | chirales Zentrum; |
| $R^1$ | ein geradkettiger oder verzweigter Alkylrest mit 1 bis 22 bzw. 3 bis 22 C-Atomen, wobei auch eine oder zwei nicht benachbarte $CH_2$-Gruppen durch -O-, -CO-, -CO-O-, -O-CO-, -O-CO-O- oder $-Si(CH_3)_2$- ersetzt sein können; |
| $R^2$,$R^3$,$R^4$ | gleich oder verschieden, H oder ein geradkettiger oder verzweigter Alkylrest mit 1 bis 16 C-Atomen; |
| $A^1$, $A^2$, $A^3$ | gleich oder verschieden, 1,4-Phenylen, wobei ein, zwei oder drei H-Atome durch F ersetzt sein können, Pyridin-2,5-diyl, wobei ein oder zwei H-Atome durch F ersetzt sein können, Pyrimidin-2,5-diyl, wobei ein oder zwei H-Atome durch F ersetzt sein können, trans-1,4-Cyclohexylen, bei dem ein oder zwei H-Atome durch - CN und/oder $-CH_3$ ersetzt sein können, (1,3,4)-Thiadiazol-2,5-diyl; |
| $M^1$, $M^2$ | gleich oder verschieden, -CO-O-, -O-CO-, $-CH_2-O-$, $-O-CH_2-$, $-CH_2-CH_2-$; |
| a, b, c, d, e | null oder eins. |

L. Optisch aktive Dioxolanether der Formel (XIV),

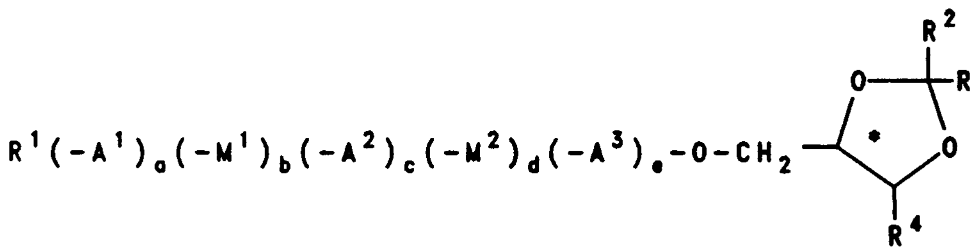

(XIV)

wobei die Symbole und Indizes folgende Bedeutung haben:

| | |
|---|---|
| * | chirales Zentrum; |
| $R^1$ | ein geradkettiger oder verzweigter Alkylrest mit 1 bis 22 bzw. 3 bis 22 C-Atomen, wobei auch eine oder zwei nicht benachbarte $CH_2$-Gruppen durch -O-, -CO-, -CO-O-, -O-CO-, -O-CO-O- oder $-Si(CH_3)_2-$ ersetzt sein können; |
| $R^2$, $R^3$,$R^4$ | gleich oder verschieden, H oder ein geradkettiger oder verzweigter Alkylrest mit 1 bis 16 C-Atomen, wobei $R^2$ und $R^3$ zusammen auch $-(CH_2)_5$ oder $-(CH_2)_4-$ sein können; |
| $A^1$, $A^2$, $A^3$, | gleich oder verschieden 1,4-Phenylen, wobei ein, zwei oder drei H-Atome durch F ersetzt sein können, Pyridin-2,5-diyl, wobei ein oder zwei H-Atome durch F ersetzt sein können, Pyrimidin-2,5-diyl, wobei ein oder zwei H-Atome durch F ersetzt sein können, trans-1,4-Cyclohexylen, bei dem ein oder zwei H-Atome durch - CN und/oder $-CH_3$ ersetzt sein können, (1,3,4)-Thiadiazol-2,5-diyl; |
| $M^1$, $M^2$ | gleich oder verschieden, -CO-O-, -O-CO-, $-CH_2-O-$, $-O-CH_2-$, $-CH_2-CH_2-$; |
| a, b, c, d, e | null oder eins. |

M. Optisch aktive Dioxolanester der Formel (XV)

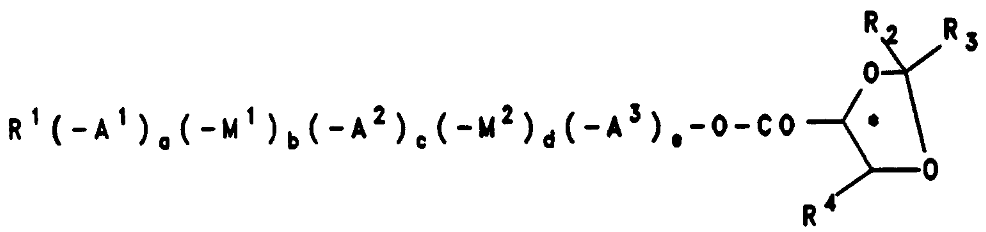

(XV)

in der bedeuten:

| | |
|---|---|
| $R^1$ | geradkettiger oder verzweigter Alkyrest mit 1 bis 16 C-Atomen, wobei eine oder mehrere nicht benachbarte $-CH_2$ Gruppen durch -O-, -CO-, -O-CO- oder -CO-O- ersetzt sein können; |
| $R^2$, $R^3$, $R^4$ | gleich oder verschieden, H oder ein geradkettiger Alkylrest mit 1 bis 16 C-Atomen, wobei $R^2$ und $R^3$ zusammen auch $-(CH_2)_5$-oder $-(CH_2)_4-$, sein können; |
| $A^1$, $A^2$, $A^3$ | gleich oder verschieden 1,4-Phenylen, wobei ein, zwei oder drei H-Atome durch F ersetzt sein können, Pyridin-2,5-diyl, wobei ein oder zwei H-Atome durch F ersetzt sein können, Pyrimidin-2,5-diyl, wobei ein oder zwei H-Atome durch F ersetzt sein können, trans-1,4-Cyclohexylen, bei dem ein oder zwei H-Atome durch - CN und/oder $-CH_3$ ersetzt sein können, (1,3,4)-Thiadiazol-2,5-diyl; |
| $M^1$, $M^2$ | gleich oder verschieden, -CO-O-, -O-CO-, $-CH_2-O-$, $-O-CH_2-$, $-CH_2-CH_2-$; |
| a, b, c, d, e | null oder eins. |

N. Makrocyclische Verbindungen der Formel (XVI),

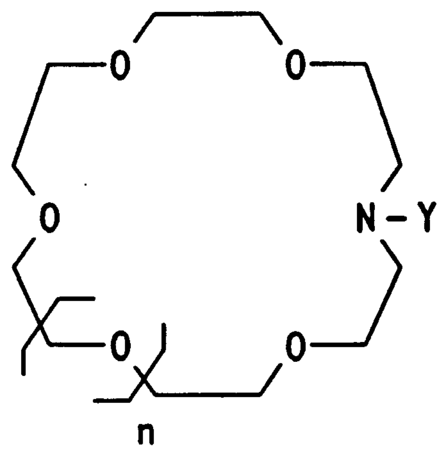

(XVI)

mit:

n : 0, 1;

Y : -CO-(t-Butyl), -CO-(Adamantyl).

Die erfindungsgemäßen Mischungen eignen sich zur Anwendung in allen Bereichen der Optotechnik, beispielsweise in Schalt- und Anzeigeelementen, Lichtventilen und Bauteilen mit NLO-Eigenschaften. Insbesondere eignen sich die erfindungsgemäßen Mischungen dort zum Einsatz, wo die Eigenschaften smektischer Flüssigkristalle genutzt werden.

Achirale und chirale Basismischungen können in allen Gebieten, in denen anisotrope Fluide eingesetzt werden, Verwendung finden, beispielsweise als Säulenmaterial für die Gaschromatographie.

Flüssigkristalline Mischungen, die Verbindungen der allgemeinen Formel (I) enthalten, sind besonders für die Verwendung in elektrooptischen Schalt-und Anzeigevorrichtungen (Displays) geeignet. Diese Displays sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z.B. aus Glas) sind. Darüberhinaus enthalten sie Abstandshalter, Kleberahmen, Polarisatoren sowie für Farbdisplays dünne Farbfilterschichten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z.B. E. Kaneko, "Liquid Crystal TV Displays: Principles and Applications of Liquid Crystal Displays", KTK Scientific Publishers 1987).

Erfindungsgemäße ferroelektrische Mischungen werden bevorzugt in den eingangs beschriebenen FLC-Zellen verwendet, die auf Ausnutzung des SSFLC-Effektes (siehe z. B. J. W. Goodby et al., Ferroelectric Liquid Crystals S. 24 ff, Gordon & Breach, Philadelphia 1991) beruhen.

In solchen Zellen werden die erfindungsgemäßen Mischungen bevorzugt in Kombinationen mit Orientierungsschichten eingesetzt, wie sie beispielsweise in DE-A 42 12 893 oder in der deutschen Patentanmeldung P 43 05 970 mit dem Titel "Cyclische Strukturelemente enthaltende Silan-Koppler als Orientierungsschichten" vorgeschlagen werden. Dort ist ein Orientierungsfilm für Flüssigkristalle beschrieben, bestehend aus einer quasi monomolekularen Schicht von Verbindungen der Formel

$C_y$ -$S_p$ - $A_n$

worin $C_y$ und $S_p$ bedeuten:

$C_y$ einen medio- oder makrocyclischen Kohlenstoffring mit 8 oder mehr Ringgliedern, wobei dieser Ring auch anellierte Benzolringe und - O-, -N-, -S-, -Si- und -B- als Heteroatome enthalten kann;

$S_p$ eine Alkylgruppe mit 1 bis 20 Kohlenstoffatomen, bei der eine oder mehrere nicht benachbarte $CH_2$-Gruppen durch -O-, -S-, -CO-, -O-CO-, -NH-CO-, -O-COO-, -NH-CO-NH-, -NH-CO-O-, $-SO_2-$, $-Si(CH_3)_2-$, -CH = CH-oder -C≡C- ersetzt sein können;

$A_n$ Si $X^1X^2X^3$, wobei

$X^1$ eine Einfachbindung und

$X^2$, $X^3$ gleich oder verschieden eine Einfachbindung, eine Alkyl oder eine Alkoxygruppe ist, und

wobei die Verbindungen über die Einfachbindung(en) der Gruppe $A_n$ an eine sauerstoffhaltige Schicht

gebunden sind.

Ferner sind erfindungsgemäß Mischungen für Feldbehandlung, d. h. zum Betrieb in der Quasi-Bookshelf-Geometrie (QBG), (siehe z. B. H. Rieger et al., SID 91 Digest (Anaheim) 1991, p. 396), geeignet.

Ebenso sind die erfindungsgemäßen Mischungen geeignet für die Verwendung in ferroelektrischen Flüssigkristallanzeigen, die auf Nutzung des DHF-Effekts oder des PSFLCD-Effekts (Pitch Stabilized Ferroelectric Liquid Crystal Display, auch SBF = Short Pitch Bistable Ferroelectric Effect genannt) beruhen.

Die Erfindung wird durch die Beispiele näher erläutert, ohne sie darauf beschränken zu wollen.

Beispiele

Die Phasenumwandlungstemperaturen werden beim Aufheizen mit Hilfe eines Polarisationsmikroskops anhand der Texturänderungen bestimmt. Die Bestimmung des Schmelzpunkts wird hingegen mit einem DSC-Gerät durchgeführt. Die Angabe der Phasenumwandlungstemperaturen zwischen den Phasen
Isotrop (I)
Nematisch (N bzw. N*)
Smektisch-C ($S_C$ bzw. $S_C^*$)
Smektisch-A ($S_A$)
Kristallin (X)
Glasübergang (Tg)
erfolgt in °C und die Werte stehen zwischen den Phasenbezeichnungen in der Phasenfolge.

Beispiel 1:

5-(2-Octyl-benzo[1,3]dioxol-5-yl)-2-octyloxy-pyridin

5 mmol 2-Octyl-benzo[1,3]dioxol-5-boronsäure und 5 mmol 5-Brom-2-octyloxy-pyridin werden in einem Gemisch aus 30 ml Toluol, 15 ml Ethanol und 15 ml Wasser gegeben. Man fügt 0,05 mmol Tetrakis-(triphenylphosphin)palladium(0) und 12 mmol Natriumcarbonat zu und erhitzt 6 Stunden zum Rückfluß. Die Phasen werden getrennt, die wässrige Phase mit tert.-Butyl-methylether extrahiert und die gesammelten organischen Phasen mit $Na_2SO_4$ getrocknet. Zur weiteren Aufreinigung wird die Substanz an Kieselgel chromatographiert.

Analog werden folgende Verbindungen hergestellt:

Beispiel 2:

5-(2-Heptyl-benzo[1,3]dioxol-5-yl)-2-heptyl-pyridin

Beispiel 3:

5-(2-Heptyl-benzo[1,3]dioxol-5-yl)-2-(6-cyclopropylhexyloxy)-pyridin

Beispiel 4:

5-(2-Heptyl-benzo[1,3]dioxol-5-yl)-2-(9-cyclopropylnonyl)-pyridin

Beispiel 5:

5-(2-Heptyl-benzo[1,3]dioxol-5-yl)-2-(perfluoro-1H,1H-heptyloxy)-pyridin

Beispiel 6:

5-(2-Heptyl-benzo[1,3]dioxol-5-yl)-2-(5-oxa-nonyloxy)-pyridin

Beispiel 7:

5-(2-Heptyl-benzo[1,3]dioxol-5-yl)-2-(5-oxa-undecyl)-pyridin

Beispiel 8:

5-(2-Heptyl-benzo[1,3]dioxol-5-yl)-2-(6-dimethylsila)decyloxy-pyridin

Beispiel 9:

5-(2-Heptyl-benzo[1,3]dioxol-5-yl)-2-(9-dimethylsila)tetradecyl-pyridin

Beispiel 10:

5-(2-Heptyl-benzo[1,3]dioxol-5-yl)-2-(1-hexansäureester)-pyridin

Beispiel 11:

5-(2-(9-Cyclopropylnonyl)-benzo[1,3]dioxol-5-yl)-2-octyloxy-pyridin

Beispiel 12:

5-(2-(9-Cyclopropylnonyl)-benzo[1,3]dioxol-5-yl)-2-heptyl-pyridin

Beispiel 13:

5-(2-(9-Cyclopropylnonyl)-benzo[1,3]dioxol-5-yl)-2-(6-cyclopropylhexyloxy)-pyridin

Beispiel 14:

5-(2-(9-Cyclopropylnonyl)-benzo[1,3]dioxol-5-yl)-2-(9-cyclopropylnonyl)-pyridin

Beispiel 15:

5-(2-(9-Cyclopropylnonyl)-benzo[1,3]dioxol-5-yl)-2-(perfluoro-1H,1H-heptyloxy)-pyridin

Beispiel 16:

5-(2-(9-Cyclopropylnonyl)-benzo[1,3]dioxol-5-yl)-2-(5-oxa-nonyloxy)-pyridin

Beispiel 17:

5-(2-(9-Cyclopropylnonyl)-benzo[1,3]dioxol-5-yl)-2-(5-oxa-undecyl)-pyridin

Beispiel 18:

5-(2-(9-Cyclopropylnonyl)-benzo[1,3]dioxol-5-yl)-2-(6-dimethylsila)decyloxy-pyridin

Beispiel 19:

5-(2-(9-Cyclopropylnonyl)-benzo[1,3]dioxol-5-yl)-2-(9-dimethylsila)tetradecyl-pyridin

Beispiel 20:

5-(2-(9-Cyclopropylnonyl)-benzo[1,3]dioxol-5-yl)-2-(1-hexansäureester)-pyridin

Beispiel 21:

5-(2-(Perfluoro-1H,1H-heptyl)-benzo[1,3]dioxol-5-yl)-2-octyloxy-pyridin

Beispiel 22:

5-(2-(Perfluoro-1H,1H-heptyl)-benzo[1,3]dioxol-5-yl)-2-heptyl-pyridin

Beispiel 23:

5-(2-(Perfluoro-1H,1H-heptyl)-benzo[1,3]dioxol-5-yl)-2-(6-cyclopropylhexyloxy)-pyridin

Beispiel 24:

5-(2-(Perfluoro-1H,1H-heptyl)-benzo[1,3]dioxol-5-yl)-2-(9-cyclopropylnonyl)-pyridin

Beispiel 25:

5-(2-(Perfluoro-1H,1H-heptyl)-benzo[1,3]dioxol-5-yl)-2-(perfluoro-1H,1H-heptyloxy)-pyridin

Beispiel 26: 5-(2-(Perfluoro-1H,1H-hep

tyl)-benzo[1,3]dioxol-5-yl)-2-(5-oxa-nonyloxy)-pyridin

Beispiel 27:

5-(2-(Perfluoro-1H,1H-heptyl)-benzo[1,3]dioxol-5-yl)-2-(5-oxa-undecyl)-pyridin

Beispiel 28:

5-(2-(Perfluoro-1H,1H-heptyl)-benzo[1,3]dioxol-5-yl)-2-(6-dimethylsila)decyloxy-pyridin

Beispiel 29:

5-(2-(Perfluoro-1H,1H-heptyl)-benzo[1,3]dioxol-5-yl)-2-(9-dimethylsila)tetradecyl-pyridin

Beispiel 30:

5-(2-(Perfluoro-1H,1H-heptyl)-benzo[1,3]dioxol-5-yl)-2-(1-hexansäureester)-pyridin

Beispiel 31:

5-(2-(5-Oxa-undecyl)-benzo[1,3]dioxol-5-yl)-2-octyloxy-pyridin

Beispiel 32:

5-(2-(5-Oxa-undecyl)-benzo[1,3]dioxol-5-yl)-2-heptyl-pyridin

Beispiel 33:

5-(2-(5-Oxa-undecyl)-benzo[1,3]dioxol-5-yl)-2-(6-cyclopropylhexyloxy)-pyridin

Beispiel 34:

5-(2-(5-Oxa-undecyl)-benzo[1,3]dioxol-5-yl)-2-(9-cyclopropylnonyl)-pyridin

Beispiel 35:

5-(2-(5-Oxa-undecyl)-benzo[1,3]dioxol-5-yl)-2-(perfluoro-1H,1H-heptyloxy)-pyridin

Beispiel 36:

5-(2-(5-Oxa-undecyl)-benzo[1,3]dioxol-5-yl)-2-(5-oxa-nonyloxy)-pyridin

Beispiel 37:

5-(2-(5-Oxa-undecyl)-benzo[1,3]dioxol-5-yl)-2-(5-oxa-undecyl)-pyridin

Beispiel 38:

5-(2-(5-Oxa-undecyl)-benzo[1,3]dioxol-5-yl)-2-(6-dimethylsila)decyloxy-pyridin

Beispiel 39:

5-(2-(5-Oxa-undecyl)-benzo[1,3]dioxol-5-yl)-2-(9-dimethylsila)tetradecyl-pyridin

Beispiel 40:

5-(2-(5-Oxa-undecyl)-benzo[1,3]dioxol-5-yl)-2-(1-hexansäureester)-pyridin

Beispiel 41:

5-(2-(9-Dimethylsila)tetradecyl-benzo[1,3]dioxol-5-yl)-2-octyloxy-pyridin

Beispiel 42:

5-(2-(9-Dimethylsila)tetradecyl-benzo[1,3]dioxol-5-yl)-2-heptyl-pyridin

Beispiel 43:

5-(2-(9-Dimethylsila)tetradecyl-benzo[1,3]dioxol-5-yl)-2-(6-cyclopropylhexyloxy)-pyridin

Beispiel 44:

5-(2-(9-Dimethylsila)tetradecyl-benzo[1,3]dioxol-5-yl)-2-(9-cyclopropylnonyl)-pyridin

Beispiel 45:

5-(2-(9-Dimethylsila)tetradecyl-benzo[1,3]dioxol-5-yl)-2-(perfluoro-1H,1H-heptyloxy)-pyridin

Beispiel 46:

5-(2-(9-Dimethylsila)tetradecyl-benzo[1,3]dioxol-5-yl)-2-(5-oxa-nonyloxy)-pyridin

Beispiel 47:

5-(2-(9-Dimethylsila)tetradecyl-benzo[1,3]dioxol-5-yl)-2-(5-oxa-undecyl)-pyridin

Beispiel 48:

5-(2-(9-Dimethylsila)tetradecyl-benzo[1,3]dioxol-5-yl)-2-(6-dimethylsila)decyloxy-pyridin

Beispiel 49:

5-(2-(9-Dimethylsila)tetradecyl-benzo[1,3]dioxol-5-yl)-2-(9-dimethylsila)tetradecyl-pyridin

Beispiel 50:

5-(2-(9-Dimethylsila)tetradecyl-benzo[1,3]dioxol-5-yl)-2-(1-hexansäureester)-pyridin

Beispiel 51:

2-(2-Heptyl-benzol[1,3]dioxol-5-yl)-5-(4-octyloxy-phenyl)pyrimidin

Beispiel 52:

2-(2-Heptyl-benzol[1,3]dioxol-5-yl)-5-(4-heptyl-phenyl)pyrimidin

Beispiel 53:

2-(2-Heptyl-benzol[1,3]dioxol-5-yl)-5-(4-(6-cyclopropylhexyloxy)-phenyl)pyrimidin

Beispiel 54:

2-(2-Heptyl-benzol[1,3]dioxol-5-yl)-5-(4-(9-cyclopropylnonyl)-phenyl)pyrimidin

Beispiel 55:

2-(2-Heptyl-benzol[1,3]dioxol-5-yl)-5-(4-(perfluoro-1H,1H-heptyloxy)-phenyl)pyrimidin

Beispiel 56:

2-(2-Heptyl-benzol[1,3]dioxol-5-yl)-5-(4-(5-oxa-nonyloxy)-phenyl)pyrimidin

Beispiel 57:

2-(2-Heptyl-benzol[1,3]dioxol-5-yl)-5-(4-(5-oxa-undecyl)-phenyl)pyrimidin

Beispiel 58:

2-(2-Heptyl-benzol[1,3]dioxol-5-yl)-5-(4-(6-dimethylsila)decyloxy-phenyl)pyrimidin

Beispiel 59:

2-(2-Heptyl-benzol[1,3]dioxol-5-yl)-5-(4-(9-dimethylsila)tetradecyl-phenyl)pyrimidin

Beispiel 60:

2-(2-Heptyl-benzol[1,3]dioxol-5-yl)-5-(4-(1-hexansäureester)-phenyl)pyrimidin

Beispiel 61:

2-(2-(9-Cyclopropylnonyl)-benzol[1,3]dioxol-5-yl)-5-(4-octyloxy-phenyl)pyrimidin

Beispiel 62:

2-(2-(9-Cyclopropylnonyl)-benzol[1,3]dioxol-5-yl)-5-(4-heptyl-phenyl)pyrimidin

Beispiel 63:

2-(2-(9-Cyclopropylnonyl)-benzol[1,3]dioxol-5-yl)-5-(4-(6-cyclopropylhexyloxy)-phenyl)pyrimidin

Beispiel 64:

2-(2-(9-Cyclopropylnonyl)-benzol[1,3]dioxol-5-yl)-5-(4-(9-cyclopropylnonyl)-phenyl)pyrimidin

Beispiel 65:

2-(2-(9-Cyclopropylnonyl)-benzol[1,3]dioxol-5-yl)-5-(4-(perfluoro-1H,1H-heptyloxy)-phenyl)pyrimidin

Beispiel 66:

2-(2-(9-Cyclopropylnonyl)-benzol[1,3]dioxol-5-yl)-5-(4-(5-oxa-nonyloxy)-phenyl)pyrimidin

Beispiel 67:

2-(2-(9-Cyclopropylnonyl)-benzol[1,3]dioxol-5-yl)-5-(4-(5-oxa-undecyl)-phenyl)pyrimidin

Beispiel 68:

2-(2-(9-Cyclopropylnonyl)-benzol[1,3]dioxol-5-yl)-5-(4-(6-dimethylsila)decyloxy-phenyl)pyrimidin

Beispiel 69:

2-(2-(9-Cyclopropylnonyl)-benzol[1,3]dioxol-5-yl)-5-(4-(9-dimethylsila)tetradecyl-phenyl)pyrimidin

Beispiel 70:

2-(2-(9-Cyclopropylnonyl)-benzol[1,3]dioxol-5-yl)-5-(4-(1-hexansäureester)-phenyl)pyrimidin

Beispiel 71:

2-(2-(Perfluoro-1H,1H-heptyl)-benzol[1,3]dioxol-5-yl)-5-(4-octyloxy-phenyl)pyrimidin

Beispiel 72:

2-(2-(Perfluoro-1H,1H-heptyl)-benzol[1,3]dioxol-5-yl)-5-(4-heptyl-phenyl)pyrimidin

Beispiel 73:

2-(2-(Perfluoro-1H,1H-heptyl)-benzol[1,3]dioxol-5-yl)-5-(4-(6-cyclopropylhexyloxy)-phenyl)pyrimidin

Beispiel 74:

2-(2-(Perfluoro-1H,1H-heptyl)-benzol[1,3]dioxol-5-yl)-5-(4-(9-cyclopropylnonyl)-phenyl)pyrimidin

Beispiel 75:

2-(2-(Perfluoro-1H,1H-heptyl)-benzol[1,3]dioxol-5-yl)-5-(4-(perfluoro-1H,1H-heptyloxy)-phenyl)pyrimidin

Beispiel 76:

2-(2-(Perfluoro-1H,1H-heptyl)-benzol[1,3]dioxol-5-yl)-5-(4-(5-oxa-nonyloxy)-phenyl)pyrimidin

Beispiel 77:

2-(2-(Perfluoro-1H,1H-heptyl)-benzol[1,3]dioxol-5-yl)-5-(4-(5-oxa-undecyl)-phenyl)pyrimidin

Beispiel 78:

2-(2-(Perfluoro-1H,1H-heptyl)-benzol[1,3]dioxol-5-yl)-5-(4-(6-dimethylsila)decyloxy-phenyl)pyrimidin

Beispiel 79:

2-(2-(Perfluoro-1H,1H-heptyl)-benzol[1,3]dioxol-5-yl)-5-(4-(9-dimethylsila)tetradecyl-phenyl)pyrimidin

Beispiel 80:

2-(2-(Perfluoro-1H,1H-heptyl)-benzol[1,3]dioxol-5-yl)-5-(4-(1-hexansäureester)-phenyl)pyrimidin

Beispiel 81:

2-(2-(5-Oxa-undecyl)-benzol[1,3]dioxol-5-yl)-5-(4-octyloxy-phenyl)pyrimidin

Beispiel 82:

2-(2-(5-Oxa-undecyl)-benzol[1,3]dioxol-5-yl)-5-(4-heptyl-phenyl)pyrimidin

Beispiel 83:

2-(2-(5-Oxa-undecyl)-benzol[1,3]dioxol-5-yl)-5-(4-(6-cyclopropylhexyloxy)-phenyl)pyrimidin

Beispiel 84:

2-(2-(5-Oxa-undecyl)-benzol[1,3]dioxol-5-yl)-5-(4-(9-cyclopropylnonyl)-phenyl)pyrimidin

Beispiel 85:

2-(2-(5-Oxa-undecyl)-benzol[1,3]dioxol-5-yl)-5-(4-(perfluoro-1H,1H-heptyloxy)-phenyl)pyrimidin

Beispiel 86:

2-(2-(5-Oxa-undecyl)-benzol[1,3]dioxol-5-yl)-5-(4-(5-oxa-nonyloxy)-phenyl)pyrimidin

Beispiel 87:

2-(2-(5-Oxa-undecyl)-benzol[1,3]dioxol-5-yl)-5-(4-(5-oxa-undecyl)-phenyl)pyrimidin

Beispiel 88:

2-(2-(5-Oxa-undecyl)-benzol[1,3]dioxol-5-yl)-5-(4-(6-dimethylsila)decyloxy-phenyl)pyrimidin

Beispiel 89:

2-(2-(5-Oxa-undecyl)-benzol[1,3]dioxol-5-yl)-5-(4-(9-dimethylsila)tetradecyl-phenyl)pyrimidin

Beispiel 90:

2-(2-(5-Oxa-undecyl)-benzol[1,3]dioxol-5-yl)-5-(4-(1-hexansäureester)-phenyl)pyrimidin

Beispiel 91:

2-(2-(9-Dimethylsila)tetradecyl-benzol[1,3]dioxol-5-yl)-5-(4-octyloxy-phenyl)pyrimidin

Beispiel 92:

2-(2-(9-Dimethylsila)tetradecyl-benzol[1,3]dioxol-5-yl)-5-(4-heptyl-phenyl)pyrimidin

Beispiel 93:

2-(2-(9-Dimethylsila)tetradecyl-benzol[1,3]dioxol-5-yl)-5-(4-(6-cyclopropylhexyloxy)-phenyl)pyrimidin

Beispiel 94:

2-(2-(9-Dimethylsila)tetradecyl-benzol[1,3]dioxol-5-yl)-5-(4-(9-cyclopropylnonyl)-phenyl)pyrimidin

Beispiel 95:

2-(2-(9-Dimethylsila)tetradecyl-benzol[1,3]dioxol-5-yl)-5-(4-(perfluoro-1H,1H-heptyloxy)-phenyl)pyrimidin

Beispiel 96:

2-(2-(9-Dimethylsila)tetradecyl-benzol[1,3]dioxol-5-yl)-5-(4-(5-oxa-nonyloxy)-phenyl)pyrimidin

Beispiel 97:

2-(2-(9-Dimethylsila)tetradecyl-benzol[1,3]dioxol-5-yl)-5-(4-(5-oxa-undecyl)-phenyl)pyrimidin

Beispiel 98:

2-(2-(9-Dimethylsila)tetradecyl-benzol[1,3]dioxol-5-yl)-5-(4-(6-dimethylsila)decyloxy-phenyl)pyrimidin

Beispiel 99:

2-(2-(9-Dimethylsila)tetradecyl-benzol[1,3]dioxol-5-yl)-5-(4-(9-dimethylsila)tetradecyl-phenyl)pyrimidin

Beispiel 100:

2-(2-(9-Dimethylsila)tetradecyl-benzol[1,3]dioxol-5-yl)-5-(4-(1-hexansäureester)-phenyl)pyrimidin

Beispiel 101:

2-(2-Heptyl-3,3,4-trifluoro-2,3-dihydro-benzofuran-5-yl)-5-(4-octyloxy-phenyl)pyrimidin

Beispiel 102:

2-(2-Heptyl-3,3,4-trifluoro-2,3-dihydro-benzofuran-5-yl)-5-(4-heptyl-phenyl)pyrimidin

Beispiel 103:

2-(2-Heptyl-3,3,4-trifluoro-2,3-dihydro-benzofuran-5-yl)-5-(4-(6-cyclopropylhexyloxy)-phenyl)pyrimidin

Beispiel 104:

2-(2-Heptyl-3,3,4-trifluoro-2,3-dihydro-benzofuran-5-yl)-5-(4-(9-cyclopropylnonyl)-phenyl)pyrimidin

Beispiel 105:

2-(2-Heptyl-3,3,4-trifluoro-2,3-dihydro-benzofuran-5-yl)-5-(4-(perfluoro-1H,1H-heptyloxy)-phenyl)pyrimidin

Beispiel 106:

2-(2-Hepty-3,3,4-trifluoro-2,3-dihydro-benzofuran-5-yl)-5-(4-(5-oxanonyloxy)-phenyl)pyrimidin

Beispiel 107:

2-(2-Hepty-3,3,4-trifluoro-2,3-dihydro-benzofuran-5-yl)-5-(4-(5-oxa-undecyl)-phenyl)pyrimidin

Beispiel 108:

2-(2-Hepty-3,3,4-trifluoro-2,3-dihydro-benzofuran-5-yl)-5-(4-(6-dimethylsila)decyloxy-phenyl)pyrimidin

Beispiel 109:

2-(2-Heptyl-3,3,4-trifluoro-2,3-dihydro-benzofuran-5-yl)-5-(4-(9-dimethylsila)tetradecyl-phenyl)pyrimidin

Beispiel 110:

2-(2-Heptyl-3,3,4-trifluoro-2,3-dihydro-benzofuran-5-yl)-5-(4-(1-hexansäureester)-phenyl)pyrimidin

Beispiel 111:

2-(2-(9-Cyclopropylnonyl)-3,3,4-trifluoro-2,3-dihydro-benzofuran-5-yl)-5-(4-octyloxy-phenyl)pyrimidin

Beispiel 112:

2-(2-(9-Cyclopropylnonyl)-3,3,4-trifluoro-2,3-dihydro-benzofuran-5-yl)-5-(4-heptyl-phenyl)pyrimidin

Beispiel 113:

2-(2-(9-Cyclopropylnonyl)-3,3,4-trifluoro-2,3-dihydro-benzofuran-5-yl)-5-(4-(6-cyclopropylhexyloxy)-phenyl)pyrimidin

Beispiel 114:

2-(2-(9-Cyclopropylnonyl)-3,3,4-trifluoro-2,3-dihydro-benzofuran-5-yl)-5-(4-(9-cyclopropylnonyl)-phenyl)-pyrimidin

Beispiel 115:

2-(2-(9-Cyclopropylnonyl)-3,3,4-trifluoro-2,3-dihydro-benzofuran-5-yl)-5-(4-(perfluoro-1H,1H-heptyloxy)-phenyl)pyrimidin

Beispiel 116:

2-(2-(9-Cyclopropylnonyl)-3,3,4-trifluoro-2,3-dihydro-benzofuran-5-yl)-5-(4-(5-oxa-nonyloxy)-phenyl)-pyrimidin

Beispiel 117:

2-(2-(9-Cyclopropylnonyl)-3,3,4-trifluoro-2,3-dihydro-benzofuran-5-yl)-5-(4-(5-oxa-undecyl)-phenyl)-pyrimidin

Beispiel 118:

2-(2-(9-Cyclopropylnonyl)-3,3,4-trifluoro-2,3-dihydro-benzofuran-5-yl)-5-(4-(6-dimethylsila)decyloxy-phenyl)pyrimidin

Beispiel 119:

2-(2-(9-Cyclopropylnonyl)-3,3,4-trifluoro-2,3-dihydro-benzofuran-5-yl)-5-(4-(9-dimethylsila)tetradecyl-phenyl)pyrimidin

Beispiel 120:

2-(2-(9-Cyclopropylnonyl)-3,3,4-trifluoro-2,3-dihydro-benzofuran-5-yl)-5-(4-(1-hexansäureester)-phenyl)-pyrimidin

Beispiel 121:

2-(2-(Perfluoro-1H,1H-heptyl)-3,3,4-trifluoro-2,3-dihydro-benzofuran-5-yl)-5-(4-octyloxy-phenyl)pyrimidin

Beispiel 122:

2-(2-(Perfluoro-1H,1H-heptyl)-3,3,4-trifluoro-2,3-dihydro-benzofuran-5-yl)-5-(4-heptyl-phenyl)pyrimidin

Beispiel 123:

2-(2-(Perfluoro-1H,1H-heptyl)-3,3,4-trifluoro-2,3-dihydro-benzofuran-5-yl)-5-(4-(6-cyclopropylhexyloxy)-phenyl)pyrimidin

Beispiel 124:

2-(2-(Perfluoro-1H,1H-heptyl)-3,3,4-trifluoro-2,3-dihydro-benzofuran-5-yl)-5-(4-(9-cyclopropylnonyl)-phenyl)pyrimidin

Beispiel 125:

2-(2-(Perfluoro-1H,1H-heptyl)-3,3,4-trifluoro-2,3-dihydro-benzofuran-5-yl)-5-(4-(perfluoro-1H,1H-heptyloxy)-phenyl)pyrimidin

Beispiel 126:

2-(2-(Perfluoro-1H,1H-heptyl)-3,3,4-trifluoro-2,3-dihydro-benzofuran-5-yl)-5-(4-(5-oxa-nonyloxy)-phenyl)-pyrimidin

Beispiel 127:

2-(2-(Perfluoro-1H,1H-heptyl)-3,3,4-trifluoro-2,3-dihydro-benzofuran-5-yl)-5-(4-(5-oxa-undecyl)-phenyl)-pyrimidin

Beispiel 128:

2-(2-(Perfluoro-1H,1H-heptyl)-3,3,4-trifluoro-2,3-dihydro-benzofuran-5-yl)-5-(4-(6-dimethylsila)decyloxy-phenyl)pyrimidin

Beispiel 129:

2-(2-(Perfluoro-1H,1H-heptyl)-3,3,4-trifluoro-2,3-dihydro-benzofuran-5-yl)-5-(4-(9-dimethylsila)tetradecyl-phenyl)pyrimidin

Beispiel 130:

2-(2-(Perfluoro-1H,1H-heptyl)-3,3,4-trifluoro-2,3-dihydro-benzofuran-5-yl)-5-(4-(1-hexansäureester)-phenyl)pyrimidin

Beispiel 131:

2-(2-(5-Oxa-undecyl)-3,3,4-trifluoro-2,3-dihydro-benzofuran-5-yl)-5-(4-octyloxy-phenyl)pyrimidin

Beispiel 132:

2-(2-(5-Oxa-undecyl)-3,3,4-trifluoro-2,3-dihydro-benzofuran-5-yl)-5-(4-heptyl-phenyl)pyrimidin

Beispiel 133:

2-(2-(5-Oxa-undecyl)-3,3,4-trifluoro-2,3-dihydro-benzofuran-5-yl)-5-(4-(6-cyclopropylhexyloxy)-phenyl)-pyrimidin

Beispiel 134:

2-(2-(5-Oxa-undecyl)-3,3,4-trifluoro-2,3-dihydro-benzofuran-5-yl)-5-(4-(9-cyclopropylnonyl)-phenyl)-pyrimidin

Beispiel 135:

2-(2-(5-Oxa-undecyl)-3,3,4-trifluoro-2,3-dihydro-benzofuran-5-yl)-5-(4-(perfluoro-1H,1H-heptyloxy)-phenyl)pyrimidin

Beispiel 136:

2-(2-(5-Oxa-undecyl)-3,3,4-trifluoro-2,3-dihydro-benzofuran-5-yl)-5-(4-(5-oxa-nonyloxy)-phenyl)pyrimidin

Beispiel 137:

2-(2-(5-Oxa-undecyl)-3,3,4-trifluoro-2,3-dihydro-benzofuran-5-yl)-5-(4-(5-oxa-undecyl)-phenyl)pyrimidin

Beispiel 138:

2-(2-(5-Oxa-undecyl)-3,3,4-trifluoro-2,3-dihydro-benzofuran-5-yl)-5-(4-(6-dimethylsila)decyloxy-phenyl)-pyrimidin

Beispiel 139:

2-(2-(5-Oxa-undecyl)-3,3,4-trifluoro-2,3-dihydro-benzofuran-5-yl)-5-(4-(9-dimethylsila)tetradecyl-phenyl)-pyrimidin

Beispiel 140:

2-(2-(5-Oxa-undecyl)-3,3,4-trifluoro-2,3-dihydro-benzofuran-5-yl)-5-(4-(1-hexansäureester)-phenyl)-pyrimidin

Beispiel 141:

2-(2-(9-Dimethylsila)tetradecyl-3,3,4-trifluoro-2,3-dihydro-benzofuran-5-yl)-5-(4-octyloxy-phenyl)pyrimidin

Beispiel 142:

2-(2-(9-Dimethylsila)tetradecyl-3,3,4-trifluoro-2,3-dihydro-benzofuran-5-yl)-5-(4-heptyl-phenyl)pyrimidin

Beispiel 143:

2-(2-(9-Dimethylsila)tetradecyl-3,3,4-trifluoro-2,3-dihydro-benzofuran-5-yl)-5-(4-(6-cyclopropylhexyloxy)-phenyl)pyrimidin

Beispiel 144:

2-(2-(9-Dimethylsila)tetradecyl-3,3,4-trifluoro-2,3-dihydro-benzofuran-5-yl)-5-(4-(9-cyclopropylnonyl)-phenyl)pyrimidin

Beispiel 145:

2-(2-(9-Dimethylsila)tetradecyl-3,3,4-trifluoro-2,3-dihydro-benzofuran-5-yl)-5-(4-(perfluoro-1H,1H-heptyloxy)-phenyl)pyrimidin

Beispiel 146:

2-(2-(9-Dimethylsila)tetradecyl-3,3,4-trifluoro-2,3-dihydro-benzofuran-5-yl)-5-(4-(5-oxa-nonyloxy)-phenyl)-pyrimidin

Beispiel 147:

2-(2-(9-Dimethylsila)tetradecyl-3,3,4-trifluoro-2,3-dihydro-benzofuran-5-yl)-5-(4-(5-oxa-undecyl)-phenyl)-pyrimidin

Beispiel 148:

2-(2-(9-Dimethylsila)tetradecyl-3,3,4-trifluoro-2,3-dihydro-benzofuran-5-yl)-5-(4-(6-dimethylsila)decyloxy-phenyl)pyrimidin

Beispiel 149:

2-(2-(9-Dimethylsila)tetradecyl-3,3,4-trifluoro-2,3-dihydro-benzofuran-5-yl)-5-(4-(9-dimethylsila)tetradecyl-phenyl)pyrimidin

Beispiel 150:

2-(2-(9-Dimethylsila)tetradecyl-3,3,4-trifluoro-2,3-dihydro-benzofuran-5-yl)-5-(4-(1-hexansäureester)-phenyl)pyrimidin

Beispiel 151:

2-Heptyl-3,3-difluoro-5-[5-(4-octyloxy-phenyl)pyrimidin-2-yl]-2,3-dihydro-furo[3,2-b]pyridin

Beispiel 152:

2-Heptyl-3,3-difluoro-5-[5-(4-heptyl-phenyl)pyrimidin-2-yl]-2,3-dihydro-furo[3,2-b]pyridin

Beispiel 153:

2-Heptyl-3,3-difluoro-5-[5-(4-(6-cyclopropylhexyloxy)-phenyl)pyrimidin-2-yl]-2,3-dihydro-furo[3,2-b]-pyridin

Beispiel 154:

2-Heptyl-3,3-difluoro-5-[5-(4-(9-cyclopropylnonyl)-phenyl)pyrimidin-2-yl]-2,3-dihydro-furo[3,2-b]pyridin

Beispiel 155:

2-Heptyl-3,3-difluoro-5-[5-(4-(perfluoro-1H,1H-heptyloxy)-phenyl)pyrimidin-2-yl]-2,3-dihydro-furo[3,2-b]-pyridin

Beispiel 156:

2-Heptyl-3,3-difluoro-5-[5-(4-(5-oxa-nonyloxy)-phenyl)pyrimidin-2-yl]-2,3-dihydro-furo[3,2-b]pyridin

Beispiel 157:

2-Heptyl-3,3-difluoro-5-[5-(4-(5-oxa-undecyl)-phenyl)pyrimidin-2-yl]-2,3-dihydro-furo[3,2-b]pyridin

Beispiel 158:

2-Heptyl-3,3-difluoro-5-[5-(4-(6-dimethylsila)decyloxy-phenyl)pyrimidin-2-yl]-2,3-dihydro-furo[3,2-b]-pyridin

Beispiel 159:

2-Heptyl-3,3-difluoro-5-[5-(4-(9-dimethylsila)tetradecyl-phenyl)pyrimidin-2-yl]-2,3-dihydro-furo[3,2-b]-pyridin

Beispiel 160:

2-Heptyl-3,3-difluoro-5-[5-(4-(1-hexansäureester)-phenyl)pyrimidin-2-yl]-2,3-dihydro-furo[3,2-b]pyridin

Beispiel 161:

2-(9-Cyclopropylnonyl)-3,3-difluoro-5-[5-(4-octyloxy-phenyl)pyrimidin-2-yl]-2,3-dihydro-furo[3,2-b]pyridin

Beispiel 162:

2-(9-Cyclopropylnonyl)-3,3-difluoro-5-[5-(4-heptyl-phenyl)pyrimidin-2-yl]-2,3-dihydro-furo[3,2-b]pyridin

Beispiel 163:

2-(9-Cyclopropylnonyl)-3,3-difluoro-5-[5-(4-(6-cyclopropylhexyloxy)-phenyl)pyrimidin-2-yl]-2,3-dihydro-furo[3,2-b]pyridin

Beispiel 164:

2-(9-Cyclopropylnonyl)-3,3-difluoro-5-[5-(4-(9-cyclopropylnonyl)-phenyl)pyrimidin-2-yl]-2,3-dihydro-furo-[3,2-b]pyridin

Beispiel 165:

2-(9-Cyclopropylnonyl)-3,3-difluoro-5-[5-(4-(perfluoro-1H,1H-heptyloxy)phenyl)pyrimidin-2-yl]-2,3-dihydro-furo[3,2-b]pyridin

Beispiel 166:

2-(9-Cyclopropylnonyl)-3,3-difluoro-5-[5-(4-(5-oxa-nonyloxy)-phenyl)pyrimidin-2-yl]-2,3-dihydro-furo[3,2-b]pyridin

Beispiel 167:

2-(9-Cyclopropylnonyl)-3,3-difluoro-5-[5-(4-(5-oxa-undecyl)-phenyl)pyrimidin-2-yl]-2,3-dihydro-furo[3,2-b]pyridin

Beispiel 168:

2-(9-Cyclopropylnonyl)-3,3-difluoro-5-[5-(4-(6-dimethylsila)decyloxy-phenyl)pyrimidin-2-yl]-2,3-dihydro-furo[3,2-b]pyridin

Beispiel 169:

2-(9-Cyclopropylnonyl)-3,3-difluoro-5-[5-(4-(9-dimethylsila)tetradecyl-phenyl)pyrimidin-2-yl]-2,3-dihydro-furo[3,2-b]pyridin

Beispiel 170:

2-(9-Cyclopropylnonyl)-3,3-difluoro-5-[5-(4-(1-hexansäureester)-phenyl)pyrimidin-2-yl]-2,3-dihydro-furo-[3,2-b]pyridin

Beispiel 171:

2-(Perfluoro-1H,1H-heptyl)-3,3-difluoro-5-[5-(4-octyloxy-phenyl)pyrimidin-2-yl]-2,3-dihydro-furo[3,2-b]-pyridin

Beispiel 172:

2-(Perfluoro-1H,1H-heptyl)-3,3-difluoro-5-[5-(4-heptyl-phenyl)pyrimidin-2-yl]-2,3-dihydro-furo[3,2-b]-pyridin

Beispiel 173:

2-(Perfluoro-1H,1H-heptyl)-3,3-difluoro-5-[5-(4-(6-cyclopropylhexyloxy)-phenyl)pyrimidin-2-yl]-2,3-dihydro-furo[3,2-b]pyridin

Beispiel 174:

2-(Perfluoro-1H,1H-heptyl)-3,3-difluoro-5-[5-(4-(9-cyclopropylnonyl)-phenyl)pyrimidin-2-yl]-2,3-dihydro-furo[3,2-b]pyridin

Beispiel 175:

2-(Perfluoro-1H,1H-heptyl)-3,3-difluoro-5-[5-(4-(perfluoro-1H,1H-heptyloxy)-phenyl)pyrimidin-2-yl]-2,3-dihydro-furo[3,2-b]pyridin

Beispiel 176:

2-(Perfluoro-1H,1H-heptyl)-3,3-difluoro-5-[5-(4-(5-oxa-nonyloxy)-phenyl)pyrimidin-2-yl]-2,3-dihydro-furo-[3,2-b]pyridin

Beispiel 177:

2-(Perfluoro-1H,1H-heptyl)-3,3-difluoro-5-[5-(4-(5-oxa-undecyl)-phenyl)pyrimidin-2-yl]-2,3-dihydro-furo-[3,2-b]pyridin

Beispiel 178:

2-(Perfluoro-1H,1H-heptyl)-3,3-difluoro-5-[5-(4-(6-dimethylsila)decyloxy-phenyl)pyrimidin-2-yl]-2,3-dihydro-furo[3,2-b]pyridin

Beispiel 179:

2-(Perfluoro-1H,1H-heptyl)-3,3-difluoro-5-[5-(4-(9-dimethylsila)tetradecyl-phenyl)pyrimidin-2-yl]-2,3-dihydro-furo[3,2-b]pyridin

Beispiel 180:

2-(Perfuoro-1H,1H-heptyl)-3,3-difluoro-5-[5-(4-(1-hexansäureester)-phenyl)pyrimidin-2-yl]-2,3-dihydro-furo[3,2-b]pyridin

Beispiel 181:

2-(5-Oxa-undecyl)-3,3-difluoro-5-[5-(4-octyloxy-phenyl)pyrimidin-2-yl]-2,3-dihydro-furo[3,2-b]pyridin

Beispiel 182:

2-(5-Oxa-undecyl)-3,3-difluoro-5-[5-(4-heptyl-phenyl)pyrimidin-2-yl]-2,3-dihydro-furo[3,2-b]pyridin

Beispiel 183:

2-(5-Oxa-undecyl)-3,3-difluoro-5-[5-(4-(6-cyclopropylhexyloxy)-phenyl)pyrimidin-2-yl]-2,3-dihydro-furo-[3,2-b]pyridin

Beispiel 184:

2-(5-Oxa-undecyl)-3,3-difluoro-5-[5-(4-(9-cyclopropylnonyl)-phenyl)pyrimidin-2-yl]-2,3-dihydro-furo[3,2-b]pyridin

Beispiel 185:

2-(5-Oxa-undecyl)-3,3-difluoro-5-[5-(4-(perfluoro-1H,1H-heptyloxy)-phenyl)pyrimidin-2-yl]-2,3-dihydro-furo[3,2-b]pyridin

Beispiel 186:

2-(5-Oxa-undecyl)-3,3-difluoro-5-[5-(4-(5-oxa-nonyloxy)-phenyl)pyrimidin-2-yl]-2,3-dihydro-furo[3,2-b]-pyridin

Beispiel 187:

2-(5-Oxa-undecyl)-3,3-difluoro-5-[5-(4-(5-oxa-undecyl)-phenyl)pyrimidin-2-yl]-2,3-dihydro-furo[3,2-b]-pyridin

Beispiel 188:

2-(5-Oxa-undecyl)-3,3-difluoro-5-[5-(4-(6-dimethylsila)decyloxy-phenyl)pyrimidin-2-yl]-2,3-dihydro-furo-[3,2-b]pyridin

Beispiel 189:

2-(5-Oxa-undecyl)-3,3-difluoro-5-[5-(4-(9-dimethylsila)tetradecyl-phenyl)pyrimidin-2-yl]-2,3-dihydro-furo-[3,2-b]pyridin

Beispiel 190:

2-(5-Oxa-undecyl)-3,3-difluoro-5-[5-(4-(1-hexansäureester)-phenyl)pyrimidin-2-yl]-2,3-dihydro-furo[3,2-b]-pyridin

Beispiel 191:

2-(9-Dimethylsila)tetradecyl-3,3-difluoro-5-[5-(4-octyloxy-phenyl)pyrimidin-2-yl]-2,3-dihydro-furo[3,2-b]-pyridin

Beispiel 192:

2-(9-Dimethylsila)tetradecyl-3,3-difluoro-5-[5-(4-heptyl-phenyl)pyrimidin-2-yl]-2,3-dihydro-furo[3,2-b]-pyridin

Beispiel 193:

2-(9-Dimethylsila)tetradecyl-3,3-difluoro-5-[5-(4-(6-cyclopropylhexyloxy)-phenyl)pyrimidin-2-yl]-2,3-dihydro-furo[3,2-b]pyridin

Beispiel 194:

2-(9-Dimethylsila)tetradecyl-3,3-difluoro-5-[5-(4-(9-cyclopropylnonyl)-phenyl)pyrimidin-2-yl]-2,3-dihydro-furo[3,2-b]pyridin

Beispiel 195:

2-(9-Dimethylsila)tetradecyl-3,3-difluoro-5-[5-(4-(perfluoro-1H,1H-heptyloxy)-phenyl)pyrimidin-2-yl]-2,3-dihydro-furo[3,2-b]pyridin

Beispiel 196:

2-(9-Dimethylsila)tetradecyl-3,3-difluoro-5-[5-(4-(5-oxa-nonyloxy)-phenyl)pyrimidin-2-yl]-2,3-dihydro-furo-[3,2-b]pyridin

Beispiel 197:

2-(9-Dimethylsila)tetradecyl-3,3-difluoro-5-[5-(4-(5-oxa-undecyl)-phenyl)pyrimidin-2-yl]-2,3-dihydro-furo-[3,2-b]pyridin

Beispiel 198:

2-(9-Dimethylsila)tetradecyl-3,3-difluoro-5-[5-(4-(6-dimethylsila)decyloxy-phenyl)pyrimidin-2-yl]-2,3-dihydro-furo[3,2-b]pyridin

Beispiel 199:

2-(9-Dimethylsila)tetradecyl-3,3-difluoro-5-[5-(4-(9-dimethylsila)tetradecyl-phenyl)pyrimidin-2-yl]-2,3-dihydro-furo[3,2-b]pyridin

Beispiel 200:

2-(9-Dimethylsila)tetradecyl-3,3-difluoro-5-[5-(4-(1-hexansäureester)-phenyl)pyrimidin-2-yl]-2,3-dihydro-furo[3,2-b]pyridin.

Beispiel 201:

Benzo[1,3]dioxol-5-carbonsäure-4-(5-octyl-pyrimidin-2-yl)-phenylester
5 mmol Benzo[1,3]dioxol-5-carbonsäure und 4,8 mmol 4-(5-octylpyrimidin-2-yl)-phenol werden in 50 ml $CH_2Cl_2$ gelöst und mit 5 mmol DCC und 0,05 mmol DMAP versetzt. Man rührt unter Lichtausschluß 18 Stunden, zieht das Lösemittel im Vakuum ab und reinigt durch Säulenchromatographie an Kieselgel. Man erhält Benzo[1,3]dioxol-5-carbonsäure-4-(5-octyl-pyrimidin-2-yl)-phenylester.
Phasenfolge:

X 94(81) N 126 I

Beispiel 202:

3-Benzo-[1,3]dioxol-5-yl-acrylsäure-4-(5-octyl-pyrimidin-2-yl)phenylester
Analog Beispiel 201 aus 3-Benzo-[1,3]dioxol-5-yl-acrylsäure und 4-(5-octylpyrimidin-2-yl)phenol.
Phasenfolge:
X 127(113) N 173 I
Analog diesem Beispiel lassen sich 2-substituierte Benzo[1,3]dioxol-5-cabonsäuren und 2-substituierte Indan-5-carbonsäuren mit substituierten Phenolen, Pyrimidinolen, Pyridinolen, Pyrazinolen, Pyridazinolen, Phenylbenzoatolen, Phenylpyrimidinolen, Phenylpyridazinolen, Phenylpyrazinolen, Phenylpyridinolen, Pyridylpyrimidinolen, Pyridylpyridazinolen, Pyridylpyrazinolen, Pyrimidinylpyrimidinolen, Pyrimidinylpyrazinolen, Pyrimidinylpyridazinolen und Pyridazinylpyrazinolen umsetzen.

Beispiel 204:

Benzo[1,3]dioxol-5-carbonsäure-4-(2-octylsulfanyl-pyrimidin-5-yl)-phenyl-ester
Phasenfolge:
X 104(75) $S_A$ 113 I

Beispiel 205:

Benzo[1,3]dioxol-5-carbonsäure-4-(5-octyloxy-pyrimidin-2-yl)-phenyl-ester
Phasenfolge:
X 116(98) N 162 I

Beispiel 206:

Benzo[1,3]dioxol-5-carbonsäure-4-(4-octyloxy-benzoyloxy)-phenyl-ester
Phasenfolge:
$X_2$ 72(84) X 110 N 177 I

Beispiel 207:

Benzo[1,3]-dioxol-5-carbonsäure-4-octyloxyphenyl-ester
Phasenfolge:
X 65 I

Beispiel 208:

5-Octyloxy-2-[4-(2-octyl-benzo[1,3]dioxol-5-yl-methoxy)-phenyl]-pyrimidin
Durch Mitsunobu-veretherung von 4-(5-Octyloxy-pyrimidin-2-yl)-phenol mit (2-Octyl-benzo[1,3]dioxol-5-yl)-methanol.

Analog diesem Beispiel lassen sich 2-substituierte Benzo[1,3]dioxol-5-yl-methanole und 2-substituierte Indan-5-carbonsäuren mit substituierten Phenolen, Pyrimidinolen, Pyridinolen, Pyrazinolen, Pyridazinolen, Phenylbenzoatolen, Phenylpyrimidinolen, Phenylpyridazinolen, Phenylpyrazinolen, Phenylpyridinolen, Pyridylpyrimidinolen, Pyridylpyridazinolen, Pyridylpyrazinolen, Pyrimidinylpyrimidinolen, Pyrimidinylpyrazinolen, Pyrimidinylpyridazinolen und Pyridazinylpyrazinolen umsetzen.

Beispiel 209:

(Benzo[1,3]-dioxol-5-yl)-2-(4-octyloxy-phenyl)-pyridin
Analog Beispiel 1 aus Benzo[1,3]dioxol-5-boronsäure und 4-(5-Brom-pyrimidin-2-yl)phenyl-octylether
Phasenfolge:
X 135 N 171 I
Analog diesem Beispiel lassen sich substituierte Benzo[1,3]dioxol-5-boronsäurederivate und substituierte Indan-5-boronsäuren mit substituierten Brombenzolen, Brompyrimidinen, Brompyridinen, Brompyrazinen, Brompyridazinen, Bromphenylbenzoaten, Bromphenylpyrimidinen, Bromphenylpyridazinen, Bromphenylpyrazinen, Bromphenylpyridinen, Brompyridylpyrimidinen, Brompyridylpyridazinen, Brompyridylpyrazinen, Brompyrimidinylpyrimidinen, Brompyrimidinylpyrazinen, Brompyrimidinylpyridazinen und Brompyridazinylpyrazinen umsetzen.

Beispiel 210:

5-{5-[2-(4-Hexyl-phenyl)-ethyl]-1,3-dioxan-2-yl}-benzo[1,3]dioxol
Durch Umsetzung von Benzo[1,3]dioxol-5-carbaldehyd mit 2-[2-(4-Hexylphenyl)-ethyl]-propan-1,3-diol unter Säurekatalyse am Wasserabscheider.
Phasenfolge:
X 89 I
Analog diesem Beispiel lassen sich substituierte Benzo[1,3]dioxol-5-carbaldehyde und substituierte Indan-5-carbaldehyde mit durch Brombenzolen, Pyrimidinen, Pyridinen, Pyrazinen, Pyridazinen, Phenylbenzoaten, Phenylpyrimidinen, Phenylpyridazinen, Phenylpyrazinen, Phenylpyridinen, Pyridylpyrimidinen, Pyridylpyridazinen, Pyridylpyrazinen, Pyrimidinpyrimidinylen, Pyrimidinylpyrazinen, Pyrimidinylpyridazinen und Pyridazinylpyrazinen substituierten 1,3-Propandiolen umsetzen.

Beispiel 211:

2-[4-(Benzo[1,3]dioxol-5-ylmethoxy)-phenyl]-5-octyloxy-pyrimidin
Duch Mitsunobu-veretherung von 4-(5-Octyloxy-pyrimidin-2-yl)-phenol mit Benzo[1,3]dioxol-5-yl-methanol analog Beispiel 208.
Phasenfolge:
X 94 N 113 I

Beispiel 212:

5-(Benzo[1,3]dioxol-5-ylmethoxy)-2-(4-octyloxy-phenyl)-pyrimidin
Duch Mitsunobu-veretherung von 2-(4-Octyloxy-phenyl)-pyrimidin-5-ol mit Benzo[1,3]dioxol-5-yl-methanol analog Beispiel 208.
Phasenfolge:
X 122 N 118 I

Beispiel 213:

2-[4-(Benzo[1,3]dioxol-5-ylmethoxy)-phenyl]-5-octyl-pyrimidin
Duch Mitsunobu-veretherung von 4-(5-Octyl-pyrimidin-2-yl)-phenol mit Benzo[1,3]dioxol-5-yl-methanol analog Beispiel 208.
Phasenfolge:
X 91 N 81 I

Anwendungsbeispiele

Eine Mischung M1, bestehend aus

| Gewichtsprozent | |
|---|---|
| 9,02 | 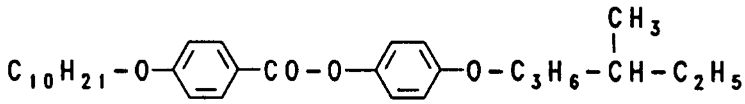 |
| 7,77 | 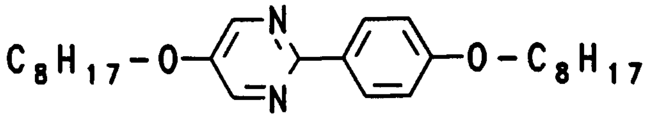 |

| Gewichtsprozent | |
|---|---|
| 3,48 | 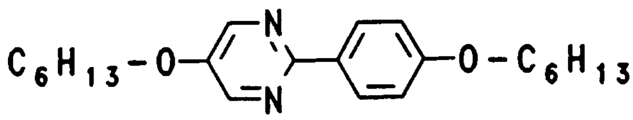 |
| 7,24 | 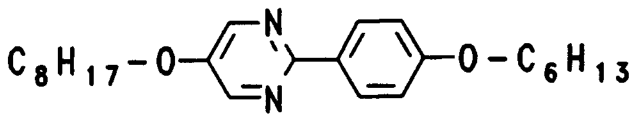 |
| 6,58 | 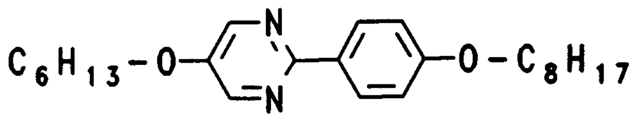 |
| 5,29 | 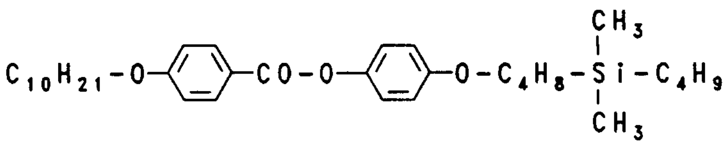 |
| 12,69 | 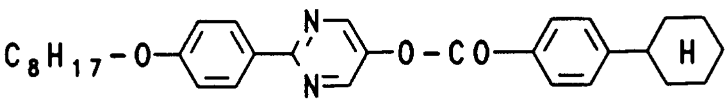 |
| 7,73 | 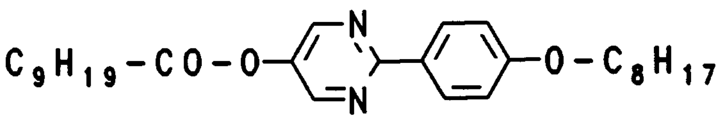 |
| 6,40 | 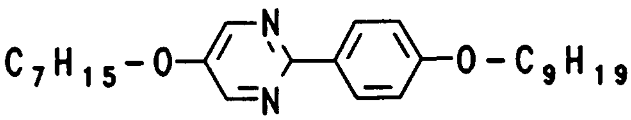 |

| Gewichtsprozent | |
|---|---|
| 3,00 | 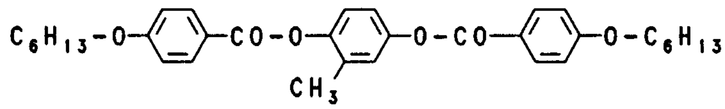 |
| 6,64 | 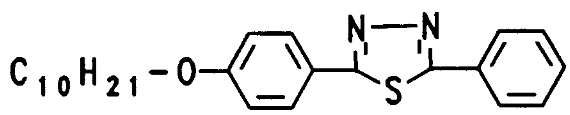 |
| 3,98 | 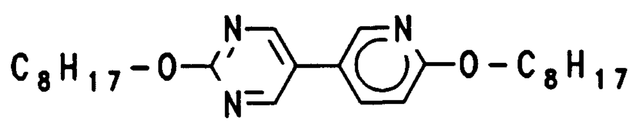 |
| 6,30 | 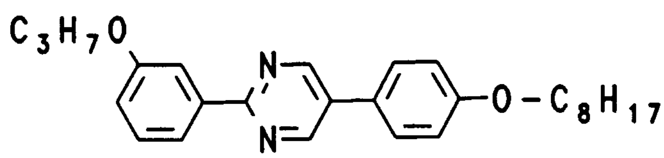 |
| 5,76 | 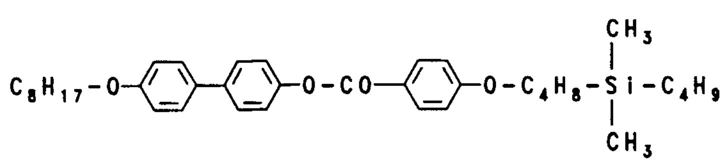 |
| 8,12 | 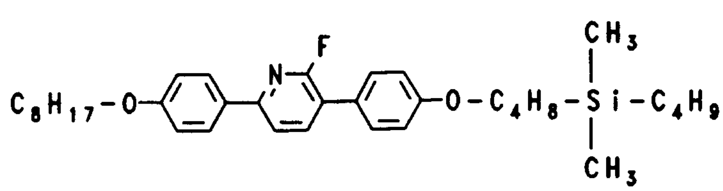 |

zeigt die Phasenfolge:

$S_C$ 79,5 ° $S_A$ 90,5 ° N 102 I

Zu dieser Mischung werden jeweils 10 Gew.-%, bezogen auf das Gewicht der gesamten Mischung, einer erfindungsgemäßen Substanz gegeben (Anwendungsbeispiele 1 bis 7) und die Phasenfolgen der resultierenden Mischungen bestimmt.

Anwendungsbeispiel 1

M1 + 10 %

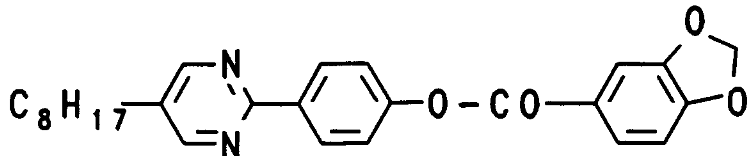

Phasenfolge: $S_C$ 73° N 102° I.

Anwendungsbeispiel 2

M1 + 10 %

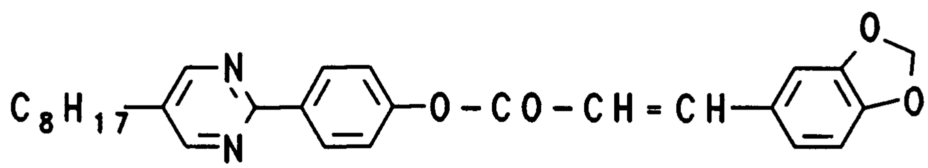

Phasenfolge: $S_C$ 72° N 105° I.

Anwendungsbeispiel 3

M1 + 10 %

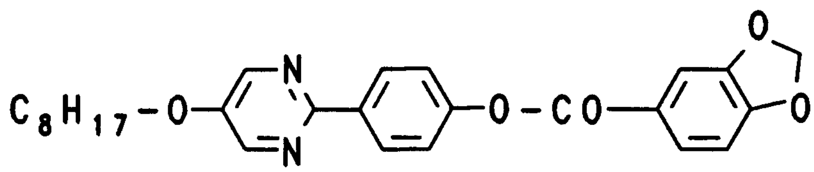

Phasenfolge: $S_C$ 75° N 106° I.

Anwendungsbeispiel 4

M1 + 10 %

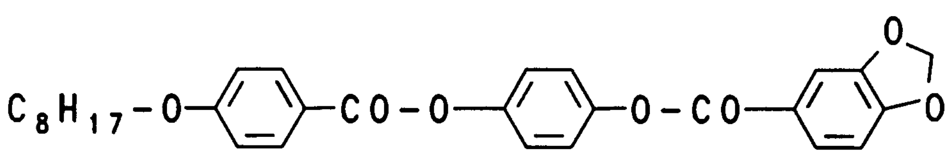

Phasenfolge: $S_C$ 75° N 105° I.

Anwendungsbeispiel 5

M1 + 10 %

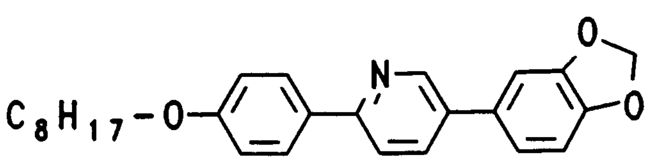

Phasenfolge: $S_C$ 79° $S_A$ 94,5° N 108 I.

Anwendungsbeispiel 6

M1 + 10 %

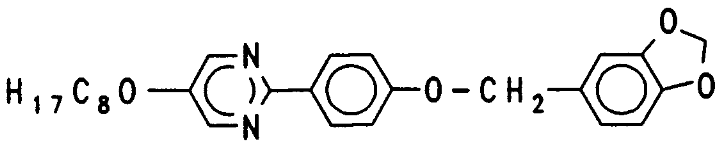

Phasenfolge: $S_C$ 81° $S_A$ 87° N 103° I.

Anwendungsbeispiel 7

M1 + 10 %

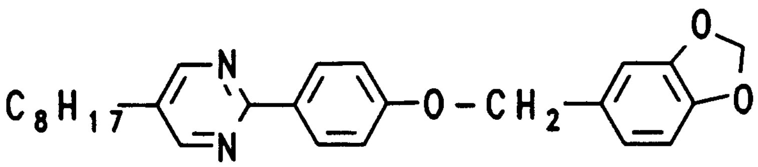

Phasenfolge: $S_C$ 76° $S_A$ 83° N 100° I.

Man erkennt, daß in allen Fällen im Vergleich zu M1 die nematische Phase verbreitert wird.

Anwendungsbeispiel 8:

Eine ferroelektrische Mischung M2 besteht aus:

| Gewichtsprozent | |
|---|---|
| 79,77 | M1 |
| 3,72 | 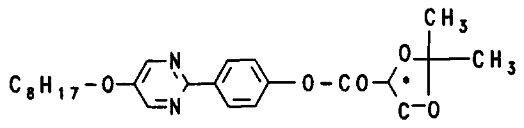 |
| 3,83 | 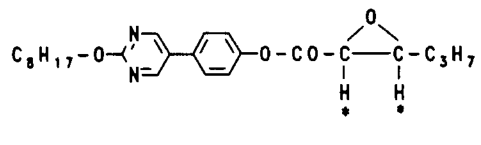 |
| 4,06 | 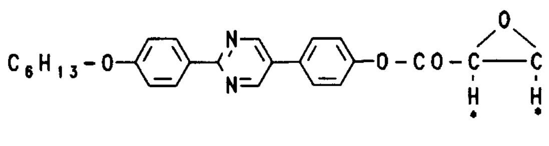 |
| 7,76 | 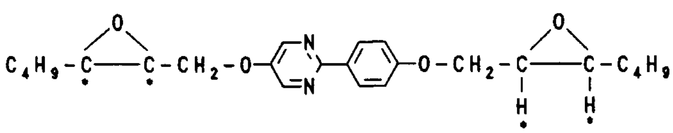 |
| 0,87 | 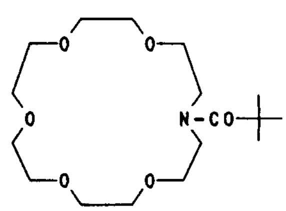 |

Zu dieser Mischung werden 10 Gew.-%, bezogen auf das Gewicht der gesamten Mischung, der erfindungsgemäßen Verbindung aus Anwendungsbeispiel 6 gegeben. Die so erhaltene Mischung zeigt eine Spontanpolarisation $P_S$ von 68,5 nC/cm$^2$ bei 25 ° C.

Anwendungsbeispiel 9

Zu der ferroelektrischen Grundmischung M2 aus Anwendungsbeispiel 8 werden 10 Gew.-%, bezogen auf das Gewicht der gesamten Mischung, der erfindungsgemäßen Verbindung aus Anwendungsbeispiel 7 gegeben. Die so erhaltene Mischung zeigt eine Spontanpolarisation $P_s$ von 66 nC/cm$^2$ bei 25 ° C.

Vergleichsbeispiel

Zu der ferroelektrischen Grundmischung M2 aus Anwendungsbeispiel 8 werden 10 Gew.-%, bezogen auf das Gewicht der gesamten Mischung, der aus dem Stand der Technik bekannten Verbindung

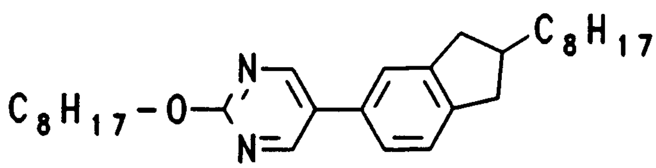

gegeben. Die so erhaltene Mischung zeigt eine Spontanpolarisation $P_s$ von nur 49 $nC/cm^2$ bei 25 °C.

Die Anwendungsbeispiele belegen, daß die erfindungsgemäßen Verbindungen zur Herstellung von smektischen bzw. ferroelektrischen Flüssigkristallmischungen geeignet sind, die eine hohe Spontanpolarisation besitzen und eine nematische Phase aufweisen, die so breit ist, daß die Orientierung der Flüssigkristallmischung beim Abkühlen günstig beeinflußt wird.

**Patentansprüche**

**1.** Aromatische Verbindung der Formel (I),

$$R^1(-A^1)_a(-M^1)_b(-A^2)_c(-M^2)_d\text{-}B(-M^3)_e(-A^3)_f(-M^4)_g(-A^4)_h\text{-}R^2 \quad \text{(I)}$$

in der die Symbole und Indizes folgende Bedeutungen haben:

B ist eine der beiden folgenden aromatischen Gruppen:

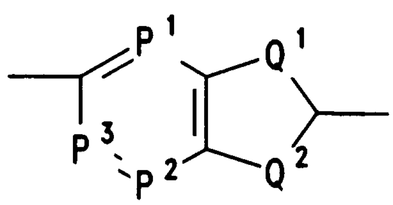

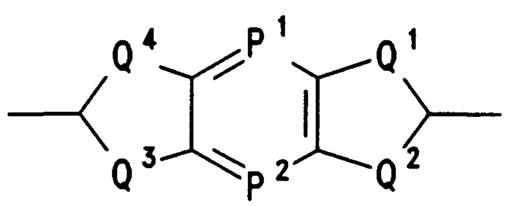

$R^1$, $R^2$ sind gleich oder verschieden Wasserstoff, - CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 20 C-Atomen (mit oder ohne asymmetrisches C-Atom), wobei auch eine oder mehrere $CH_2$-Gruppen durch -O-, -S-, -CO-, -CS-, -CH=CH-, -C≡C-, △, $-Si(CH_3)_2-$, 1,4-Phenylen, trans-1,4-Cyclohexylen oder trans-1,3-Cyclopentylen ersetzt sein können, mit der Maßgabe, daß Sauerstoffatome und Schwefelatome nicht unmittelbar miteinander gebunden sein dürfen, und/oder ein oder mehrere H-Atome des Alkylrestes durch -F, -Cl, -Br, - $OR^2$, -SCN, -OCN oder $-N_3$ substituiert sein können, oder auch eine der nachfolgenden chiralen Gruppen:

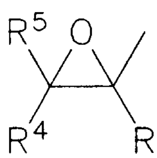
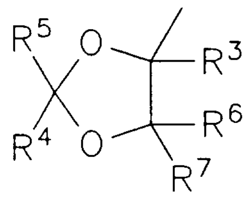
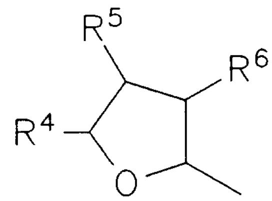

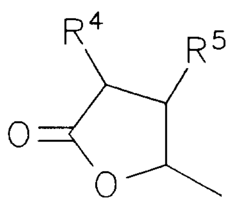
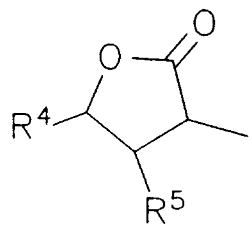
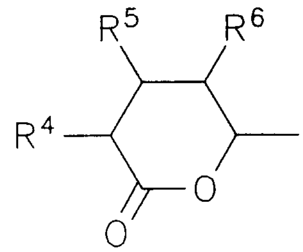

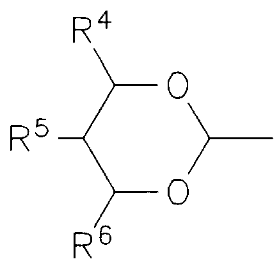
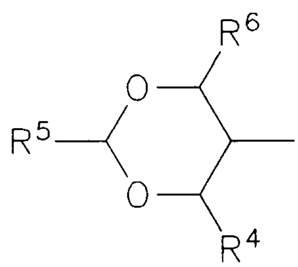
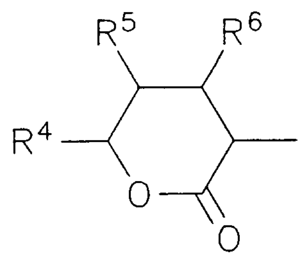

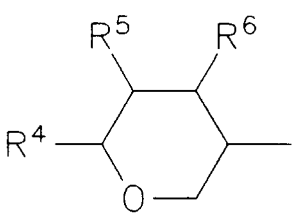
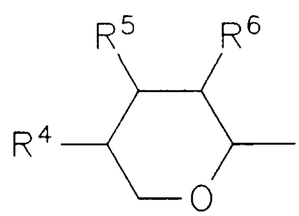
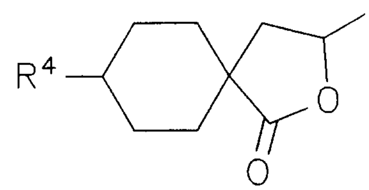

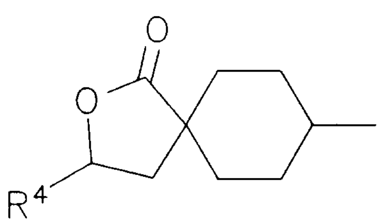
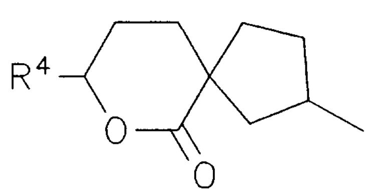
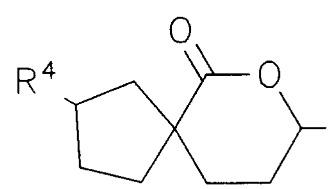

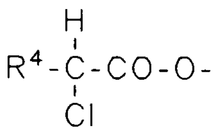 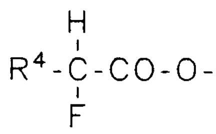 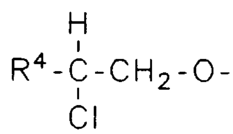 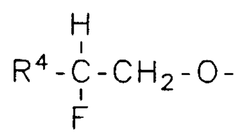

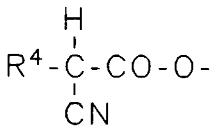 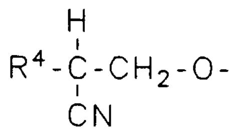 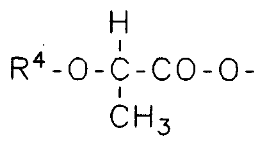 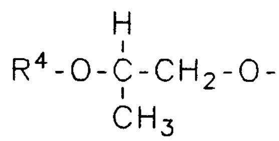

$R^3$, $R^4$, $R^5$, $R^6$, $R^7$ sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- oder -CH = CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4$- oder - $(CH_2)_5$- sein, wenn sie an ein Oxiran-, Dioxolan-, Tetrahydrofuran-, Tetrahydropyran-, Butyrolacton- oder Valerolacton-System gebunden sind;

$Q^1$, $Q^2$, $Q^3$, $Q^4$ sind gleich oder verschieden $CH_2$, $CF_2$ oder N-$R^3$, S, O,C = O;

$P^1$, $P^2$, $P^3$ sind gleich oder verschieden C-H, C-F oder N;

$M^1$, $M^2$, $M^3$, $M^4$ sind gleich oder verschieden -O-, -S-, -CO-, -CO-O-, -O-CO-, -O-CO-O-, -CO-S-, -S-CO-, -CS-O-, -O-CS-, -S-CS-S-, -O-CS-O, -S-CO-S-, -CS-, $-CH_2-O-$, $-O-CH_2-$, $-CH_2-S-$, $-S-CH_2-$, -CH = CH-, -C≡C-, -O-CO-C≡C-, -C≡C-COO-, -O-CO-CH = CH-, -CH = CH-COO-, $-O-CO-CH_2-CH_2-$, $-CH_2-CH_2-COO-$, $-O-CH_2-C≡C-$, $-C≡C-CH_2-O-$, $-O-CH_2-CH = CH-$, $-CH = CH-CH_2-O-$, $-O-CH_2-CH_2-CH_2-$, $-CH_2-CH_2-CH_2-O-$oder eine Einfachbindung;

$A^1$, $A^2$, $A^3$, $A^4$, sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Piperazin-1,4-diyl, Piperazin-2,5-diyl, Naphthalin-2,6-diyl, wobei ein oder mehrere H-Atome durch F, Cl und/oder CN ersetzt sein können, Bicyclo[2.2.2]octan-1,4-diyl, wobei ein oder mehrere H-Atome durch F, Cl und/oder CN ersetzt sein können, 1,3-Dioxaborinan-2,5-diyl oder die Gruppe B;

a, b, c, d, e, f, g, h sind null oder eins;

mit der folgenden Maßgabe:

wenn die Gruppe B

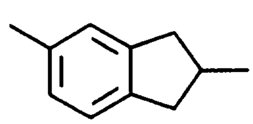

ist, muß mindestens einer der Reste $R^1$ eine $-Si(CH_3)_2-$ und/oder △ Gruppe enthalten und/oder eine

der folgenden chiralen Gruppen sein:

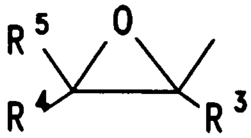 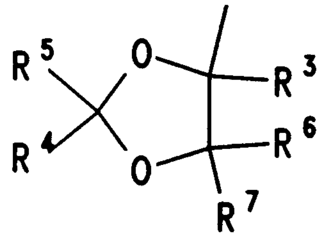

2. Aromatische Verbindung der Formel (I) nach Anspruch 1, wobei die Symbole und Indizes folgende Bedeutungen haben:

$R^1$, $R^2$ sind gleich oder verschieden Wasserstoff, - CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 18 C-Atomen (mit oder ohne asymmetrisches C-Atom), wobei auch eine oder mehrere $CH_2$-Gruppen durch -O-, -CO-, -CH=CH-, -C≡C-, △, $-Si(CH_3)_2-$ oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch -F, -Cl, $-OR^3$, -OCN oder $-N_3$ substituiert sein können, oder eine der nachfolgenden chiralen Gruppen:

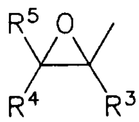 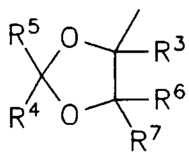 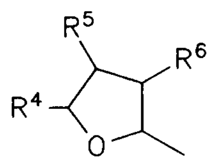

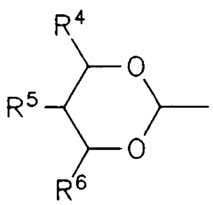 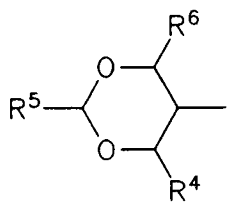 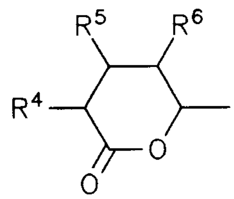

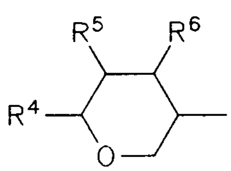 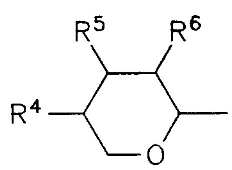 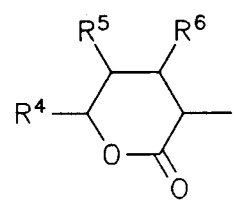

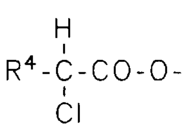 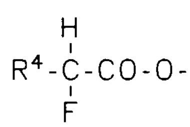 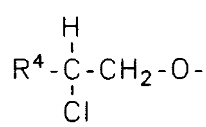 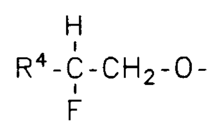

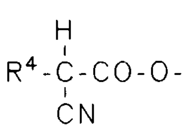 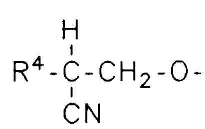 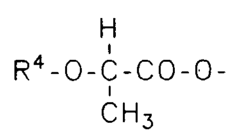 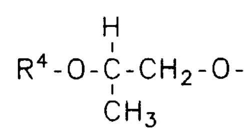

$R^3$, $R^4$, $R^5$, $R^6$, $R^7$ sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- oder -CH=CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Oxiran-, Dioxolan-, Tetrahydrofuran-, Tetrahydropyran-, Butyrolacton- oder Valerolacton-System gebunden sind;

$Q^1$, $Q^2$, $Q^3$, $Q^4$ sind gleich oder verschieden $CH_2$, $CF_2$ oder N-$R^3$, O, C=O;

$P^1$, $P^2$, $P^3$ sind gleich oder verschieden C-H, C-F oder N;

$M^1$, $M^2$, $M^3$, $M^4$ sind gleich oder verschieden -O-, -CO-, -CO-O-, -O-CO-, -O-CO-O-, -O-CS-O-, $-CH_2-O-$, $-O-CH_2-$, -CH=CH-, -C≡C-, -O-CO-C≡C-, -C≡C-COO-, -O-CO-CH=CH-, -CH=CH-COO-, $-O-CO-CH_2-CH_2-$, $-CH_2-CH_2-COO-$, $-O-CH_2-C≡C-$, $-C≡C-CH_2-O-$, $-O-CH_2-CH=CH-$, $-CH=CH-CH_2-O-$, $-O-CH_2-CH_2-CH_2-$, $-CH_2-CH_2-CH_2-O-$ oder eine Einfachbindung;

$A^1$, $A^2$, $A^3$, $A^4$, sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Naphthalin-2,6-diyl, wobei ein oder mehrere H-Atome durch F, Cl und/oder CN ersetzt sein können, 1,3-Dioxaborinan-2,5-diyl oder die Gruppe B;

a, b, c, d, e, f, g, h sind null oder eins,

wobei die Maßangabe in Anspruch 1 gilt.

**3.** Aromatische Verbindung der Formel I nach Anspruch 1 oder 2, in der die Symbole und Indizes folgende Bedeutungen haben:

$R^1$, $R^2$ sind gleich oder verschieden Wasserstoff, -CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 16 C-Atomen (mit oder ohne asymmetrisches C-Atom), wobei auch eine, zwei oder drei $CH_2$-Gruppen durch -O-, -CO-, -CH=CH-, △, $-Si(CH_3)_2-$oder trans-1,4-Cyclohexylen ersetzt sein können, mit der Maßgabe, daß Sauerstoffatome nicht unmittelbar miteinander gebunden sein dürfen, und/oder ein oder mehrere H-Atome des Alkylrestes durch -F, -Cl oder $-OR^3$ substituiert sein können, oder auch eine der nachfolgenden chiralen Gruppen:

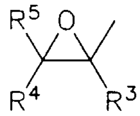 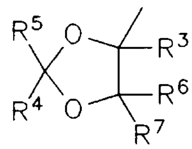 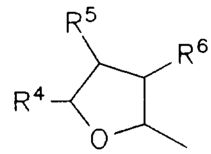

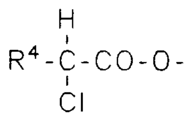 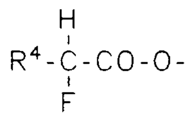 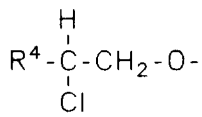 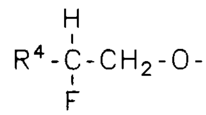

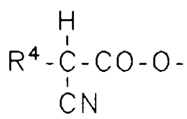 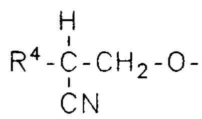 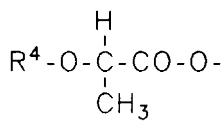 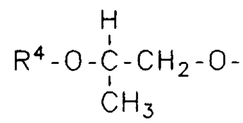

| | |
|---|---|
| $R^3$, $R^4$, $R^5$, $R^6$, $R^7$ | sind gleich oder verschieden Wasserstoff oder ein geradkettiger oder verzweigter Alkylrest mit 1-14 C-Atomen (mit oder ohne asymmetrisches C-Atom), wobei auch eine oder mehrere $CH_2$-Gruppen durch -O- oder -CH = CH- ersetzt sein können, mit der Maßgabe, daß Sauerstoffatome nicht unmittelbar miteinander gebunden sein dürfen, und/oder ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Oxiran- oder Dioxolan-System gebunden sind; |
| $Q^1$, $Q^2$, $Q^3$, $Q^4$ | sind gleich oder verschieden $CH_2$, $CF_2$ oder N-$R^3$, O, C = O; |
| $P^1$, $P^2$, $P^3$ | sind gleich oder verschieden C-H, C-F oder N; |
| $M^1$, $M^2$, $M^3$, $M^4$ | sind gleich oder verschieden -O-, -CO-, -CO-O-, -O-CO-, -O-CO-O-, $-CH_2-O-$, $-O-CH_2-$, -CH = CH-, -O-CO-C≡C-, -C≡C-COO-, -O-CO-CH = CH-, -CH = CH-COO-, $-O-CO-CH_2-CH_2-$,$-CH_2-CH_2-COO-$, $-O-CH_2-C≡C-$, $-C≡C-CH_2-O-$, $-O-CH_2-CH=CH-$, $-CH=CH-CH_2-O-$, $-O-CH_2-CH_2-CH_2-$, - $CH_2-CH_2-CH_2-O-$oder eine Einfachbindung; |
| $A^1$, $A^2$, $A^3$, $A^4$ | sind gleich oder verschieden 1,4-Phenylen, wobei ein, zwei oder drei H-Atome durch F, Cl und/oder CN ersetzt sein können, Pyrazin-2,5-diyl, wobei ein oder zwei H-Atome durch F, Cl und/oder CN ersetzt sein können,Pyridazin-3,6-diyl, wobei ein oder zwei H-Atome durch F, Cl und/oder CN ersetzt sein können,Pyridin-2,5-diyl, wobei ein oder zwei H-Atome durch F, Cl und/oder CN ersetzt sein können, Pyrimidin-2,5-diyl, wobei ein oder zwei H-Atome durch F, Cl und/oder CN ersetzt sein können,trans-1,4-Cyclohexylen, bei dem ein oder zwei H-Atome durch CN und/oder $CH_3$ ersetzt sein können, (1,3,4)-Thiadiazol-2,5-diyl, Naphthalin-2,6-diyl, wobei ein oder zwei H-Atome durch F, Cl und/oder CN ersetzt sein können,1,3-Dioxaborinan-2,5-diyl oder die Gruppe B; |
| a, b, c, d, e, f, g, h | sind null oder eins; |

mit der Maßgabe, daß mindestens einer der Reste $Q^1$, $Q^2$, $Q^3$, $Q^4$ $-CF_2-$, $-NR^3-$, -S-, -O- oder -CO- ist und/oder mindestens einer der Reste $P^1$, $P^2$, $P^3$ = CF-oder = N- ist.

**4.** Verwendung von Verbindungen der Formel I nach einem oder mehreren der Ansprüche 1 bis 3 in Flüssigkristallmischungen.

**5.** Flüssigkristallmischung, enthaltend mindestens eine Verbindung der Formel I nach einem oder mehreren der Ansprüche 1 bis 3.

6. Flüssigkristallmischung nach Anspruch 5, dadurch gekennzeichnet, daß sie ferroelektrisch ist.

7. Flüssigkristallmischung nach Anspruch 5 oder 6, dadurch gekennzeichnet, daß sie 2 bis 35 Einzelverbindungen enthält.

8. Flüssigkristallmischung nach einem oder mehreren der Ansprüche 5 bis 7, dadurch gekennzeichnet, daß sie 1 bis 10 Verbindungen der Formel I enthält.

9. Flüssigkristallmischung nach einem oder mehreren der Ansprüche 5 bis 8, dadurch gekennzeichnet, daß sie 0,01 bis 80 Gew.-% an mindestens einer Verbindung der Formel I enthält.

10. Elektrooptisches Schalt- und Anzeigeelement, enthaltend eine Flüssigkristallmischung nach einem oder mehreren der Ansprüche 5 bis 9.

Europäisches Patentamt

# EUROPÄISCHER RECHERCHENBERICHT

Nummer der Anmeldung
EP 94 11 1574

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | DE-A-39 08 269 (NEMATEL)<br>* das ganze Dokument *<br>--- | 1-10 | C07D405/00<br>C09K19/34<br>C09K19/40<br>C07F7/08<br>C07D407/00<br>C07D491/00<br>C07D317/46<br>C07D307/78 |
| X | CHEMICAL ABSTRACTS, vol. 100, no. 15, 9. April 1984, Columbus, Ohio, US;<br>abstract no. 121044,<br>'4-(Trans-4-n-Alkylcyclohexyl)phenyl piperonylates'<br>* Zusammenfassung *<br>& JP-A-83 185 579 (SUWA SEIKOSHA CO., LTD.;JAPAN ) 29. Oktober 1983<br>--- | 1-10 | |
| D,Y | EP-A-0 546 338 (CANON KK)<br>* Seite 31 - Seite 36 *<br>* Seite 152 - Seite 156 *<br>* Seite 273 - Seite 279 *<br>* Seite 378 - Seite 402 *<br>--- | 1-10 | |
| D,Y | DATABASE WPI<br>Section Ch, Week 8740,<br>Derwent Publications Ltd., London, GB;<br>Class E, AN 87-280990<br>& JP-A-62 195 355 (ASAHI GLASS) 28. August 1987<br>* Zusammenfassung *<br>--- | 1-10 | **RECHERCHIERTE SACHGEBIETE (Int.Cl.6)**<br>C07D<br>C09K |
| Y | DATABASE WPI<br>Section Ch, Week 8522,<br>Derwent Publications Ltd., London, GB;<br>Class E, AN 85-131667<br>& JP-A-60 069 055 (KANTO KAGAKU) 19. April 1985<br>* Zusammenfassung *<br>--- | 1-10 | |
| P,X | DE-A-42 18 978 (MERCK PATENT GMBH)<br>* das ganze Dokument *<br>---<br>-/-- | 1-10 | |

Der vorliegende Recherchenbericht wurde für alle Patentansprüche erstellt

| Recherchenort | Abschlußdatum der Recherche | Prüfer |
|---|---|---|
| DEN HAAG | 27. Oktober 1994 | Beslier, L |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 94 11 1574

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| P,Y | EP-A-0 555 843 (CANON KK)<br>* das ganze Dokument * | 1-10 | |
| P,Y | EP-A-0 571 955 (CANON KK)<br>* das ganze Dokument * | 1-10 | |
| E | EP-A-0 611 815 (CANON KK)<br>* das ganze Dokument * | 1-10 | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| DEN HAAG | 27. Oktober 1994 | Beslier, L |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)